# EUROPEAN PATENT SPECIFICATION

(11) **EP 1 753 417 B1**
(45) Date of publication and mention of the grant of the patent: **04.04.2012**
(21) Application number: 05758756.0
(22) Date of filing: 07.06.2005
(51) Int. Cl.: C07C 255/60

(54) **A SELECTIVE ANDROGEN RECEPTOR MODULATOR AND MEDICAL USES THEREOF**
SELEKTIVE ANDROGEN-REZEPTOR-MODULATOR UND ANWENDUNGSVERFAHREN DAFÜR
UN MODULATEUR DU RECEPTEUR D'ANDROGENE SELECTIF ET SES UTILISATIONS MEDICALES

(30) Priority: 07.06.2004 US 861923; 09.06.2004 US 863524; 12.10.2004 US 961380
(43) Date of publication of application: 21.02.2007
(60) Divisional application: 10075691.5
(73) Proprietor: UNIVERSITY OF TENNESSEE RESEARCH FOUNDATION, Knoxville, TN 37996-1527 (US)
(72) Inventor: DALTON, James, T., Upper Arlington, OH 43220 (US); MILLER, Duane, D., Germantown, TN 38139-6437 (US); VEVERKA, Karen, A., Cordova, TN 38018 (US)
(74) Representative: Lord, Hilton David
(86) International application number: PCT/US2005/019788
(87) International publication number: WO 2005/120483

(56) References cited:
- WO-A-02/16310
- WO-A-03/065992
- WO-A-03/074449
- WO-A-2005/000794
- US-A- 4 636 505
- US-A- 6 071 957
- US-A- 6 160 011
- US-A1- 2004 087 810

## Description

### BACKGROUND OF THE INVENTION

The androgen receptor ("AR") is a ligand-activated transcriptional regulatory protein that mediates induction of male sexual development and function through its activity with endogenous androgens. Androgens are generally known as the male sex hormones. The androgenic hormones are steroids which are produced in the body by the testes and the cortex of the adrenal gland or can be synthesized in the laboratory. Androgenic steroids play an important role in many physiologic processes, including the development and maintenance of male sexual characteristics such as muscle and bone mass, prostate growth, spermatogenesis, and the male hair pattern (Matsumoto, Endocrinol. Met. Clin. N. Am: 23:857-75 (1994)). The endogenous steroidal androgens include testosterone and dihydrotestosterone ("DHT"). Testosterone is the principal steroid secreted by the testes and is the primary circulating androgen found in the plasma of males. Testosterone is converted to DHT by the enzyme 5 alpha-reductase in many peripheral tissues. DHT is thus thought to serve as the intracellular mediator for most androgen actions (Zhou, et al., Molec. Endocrinol. 9:208-18 (1995)). Other steroidal androgens include esters of testosterone, such as the cypionate, propionate, phenylpropionate, cyclopentylpropionate, isocarporate, enanthate, and decanoate esters, and other synthetic androgens such as 7-Methyl-Nortestosterone ("MENT') and its acetate ester (Sundaram et al., "7 Alpha-Methyl-Nortestosterone(MENT): The Optimal Androgen For Male Contraception," Ann. Med., 25:199-205 (1993) ("Sundaram"). Because the AR is involved in male sexual development and function, the AR is a likely target for effecting male contraception or other forms of hormone replacement therapy.

BMD (bone mineral density decreases with age in both males and females. Decreased amounts of bone mineral content (BMC) and BMD correlate with decreased bone strength and predispose patients to fracture.

Osteoporosis is a systemic skeletal disease, characterized by low bone mass and deterioration of bone tissue, with a consequent increase in bone fragility and susceptibility to fracture. In the U.S., the condition affects more than 25 million people and causes more than 1.3 million fractures each year, including 500,000 spine, 250,000 hip and 240,000 wrist fractures annually. Hip fractures are the most serious consequence of osteoporosis, with 5-20% of patients dying within one year, and over 50% of survivors being incapacitated. The elderly are at greatest risk of osteoporosis, and the problem is therefore predicted to increase significantly with the aging of the population. Worldwide fracture incidence is forecasted to increase three-fold over the next 60 years, and one study estimated that there will be 4.5 million hip fractures worldwide in 2050.

Women are at greater risk of osteoporosis than men. Women experience a sharp acceleration of bone loss during the five years following menopause. Other factors that increase the risk include smoking, alcohol abuse, a sedentary lifestyle and low calcium intake. However, osteoporosis also occurs frequently in males. It is well established that the bone mineral density of males decrease with age. Decreased amounts of bone mineral content and density correlates with decreased bone strength, and predisposes to fracture. The molecular mechanisms underlying the pleiotropic effects of sex-hormones in non-reproductive tissues are only beginning to be understood, but it is clear that physiologic concentrations of androgens and estrogens play an important role in maintaining bone homeostasis throughout the life-cycle. Consequently, when androgen or estrogen deprivation occurs there is a resultant increase in the rate of bone remodeling that tilts the balance of resorption and formation to the favor of resorption that contributes to the overall loss of bone mass. In males, the natural decline in sex-hormones at maturity (direct decline in androgens as well as lower levels of estrogens derived from peripheral aromatization of androgens) is associated with the frailty of bones. This effect is also observed in males who have been castrated.

Muscle wasting refers to the progressive loss of muscle mass and/or to the progressive weakening and degeneration of muscles, including the skeletal or voluntary muscles, which control movement, cardiac muscles, which control the heart (cardiomyopathics), and smooth muscles. Chronic muscle wasting is a chronic condition (i.e. persisting over a long period of time) characterized by progressive loss of muscle mass, weakening and degeneration of muscle.

The loss of muscle mass that occurs during muscle wasting can be characterized by a muscle protein degradation by catabolism. Protein catabolism occurs because of an unusually high rate of protein degradation, an unusually low rate of protein synthesis, or a combination of both. Muscle protein catabolism, whether caused by a high degree of protein degradation or a low degree of protein synthesis, leads to a decrease in muscle mass and to muscle wasting.

Muscle wasting is associated with chronic, neurological, genetic or infectious pathologies, diseases, illnesses or conditions. These include Muscular Dystrophies such as Duchenne Muscular Dystrophy and Myotonic Dystrophy; Muscle Atrophies such as Post-Polio Muscle Atrophy (PPMA); Cachexias such as Cardiac Cachexia, AIDS Cachexia and Cancer Cachexia, malnutrition, Leprosy, Diabetes, Renal Disease, Chronic Obstructive Pulmonary Disease (COPD), Cancer, end stage Renal failure, Sarcopenia, Emphysema, Osteomalacia, HIV Infection, AIDS, and Cardiomyopathy.

In addition, other circumstances and conditions are linked to and can cause muscle, wasting. These include chronic lower back pain, advanced age, central nervous system (CNS) injury, peripheral nerve injury, spinal cord injury, chemical injury, central nervous system (CNS) damage, peripheral nerve damage, spinal cord damage, chemical damage, bums, disuse deconditioning that occurs when a limb is immobilized, long term hospitalization due to illness or injury, and alcoholism.

An intact androgen receptor (AR) signaling pathway is crucial for appropriate development of skeletal muscles. Furthermore, an intact AR-signaling pathway increases lean muscle mass, muscle strength and muscle protein synthesis.

Muscle wasting, if left unabated, can have dire health consequences. For example, the changes that occur during muscle wasting can lead to a weakened physical state that is detrimental to an individual's health, resulting in increased susceptibility to infraction and poor performance status. In addition, muscle wasting is a strong predictor of morbidity and mortality in patients suffering from cachexia and AIDS.

Innovative approaches are urgently needed at both the basic science and clinical levels to prevent and treat osteoporosis and other bone-related disorders and muscle wasting, in particular chronic muscle wasting i.e. other selective androgen receptor modulators. The present invention is directed to satisfying this need, alternative to SARM compounds known from WO 03 065992.

### SUMMARY OF THE INVENTION

The present invention provides a selective androgen receptor modulator (SARM) compound represented by a structure of formula (III):

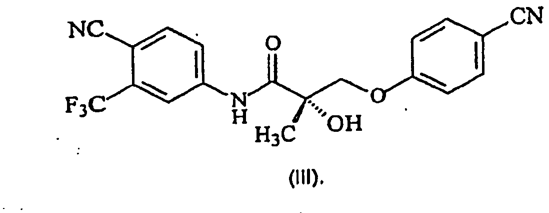

The selective androgen receptor modulator (SARM) compound according to the present invention may be an optical isomer, pharmaceutically acceptable salt, N-oxide or hydrate of the compound represented by the structure of formula (III), or, any combination thereof.

In one embodiment, the invention provides a pharmaceutical composition comprising the SARM compounds of formula (III) and a suitable carrier or diluent.

The compound of formula (III), or a composition comprising the same, may be used in treating a subject having a bone-related disorder.

The compound of formula (III) or a composition comprising the same, may be used in increasing the strength of, or mass of a bone of a subject, or in promoting bone formation in a subject.

The compound of formula (III) may be used for treating, preventing, suppressing, inhibiting or reducing the incidence of a muscle wasting disorder in a subject.

The compound of formula (III) may be used in increasing muscle performance, muscle size, muscle strength, or any combination thereof in a subject.

The compound of formula (III), or a composition comprising the same, may be used in treating obesity or diabetes associated with a metabolic syndrome in a subject.

The compound of formula (III), or a composition comprising the same, may be used in promoting or speeding recovery following a surgical procedure.

The compound of formula (III), or a composition comprising the same, may be used in promoting or suppressing spermatogenesis in a male subject.

### BRIEF DESCRIPTION OF THE DRAWINGS

**Fig 1****:** Effect of SARMs, DHT and PTH on Differentiation of Rat Bone Marrow Cells Towards Osteoblast Lineage.
**Fig 2****:** Effect of SARMs, DHT and PTH on TRAP Positive Multinucleated Osteoclasts.
**Fig 3****:** Femoral maximum load determined by 3-point bending of the femur.
**Fig 4****:** Trabecular bone mineral density determined by pQCT analysis of the distal femur.
**Fig. 5****:** Pharmacology of compound III in intact rats.
**Fig 6****:** Organ weights from castrated, compound III-treated rats presented as a percentage of intact control. * P-value < 0.05 versus intact controls.
**Fig 7****:** Organ weight maintenance dose-response curves for compound III in castrated rats. Eₘₐₓ and ED₅₀ values for the levator ani (closed triangles), prostate (open circles), and seminal vesicles (closed squares) were obtained by nonlinear regression analysis using the sigmoid Eₘₐₓ model in WinNonlin®.
**Fig 8:** Organ weights from castrated, Compound III-treated rats presented as a percentage of intact control. * P-value < 0.05 versus intact controls.
**Fig 9****:** Organ weight regrowth dose-response curves for compound III in castrated rats. Eₘₐₓ and ED₅₀ values for the levator ani (closed triangles), prostate (open circles), and seminal vesicles (closed squares) were obtained by nonlinear regression analysis using the sigmoid Eₘₐₓ model in WinNonlin®.
**Fig 10****:** Plasma concentration-time profile for compound III in healthy human volunteers with oral dose in PEG300.
**Fig 11****:** Plasma-concentration-time profiles of compound III solution vs. solid oral dosage forms.
**Fig 12****:** Plasma-concentration-time profiles of various compound III dosage forms at 30 mg.
**Fig 13****:** Dose versus AUC_{0-inf} for oral solutions (G100401)
**Fig 14:** Dose versus Cₘₐₓ for oral solutions.
**Fig 15****:** Cholesterol reduction by compound III in rats.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

In one embodiment, the invention provides a selective androgen receptor modulator (SARM) compound represented by a structure of formula (III): 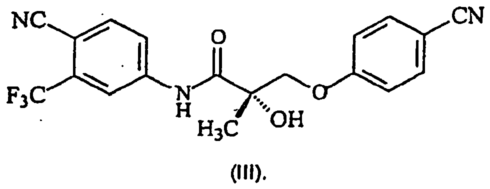 The selective androgen receptor modulator (SARM) compound according to the present invention may be an optical isomer, pharmaceutically acceptable salt, N-oxide or hydrate of the compound represented by the structure of formula (III), or any combination thereof.

In one embodiment, the invention provides a pharmaceutical composition, including compounds of formula (III) or its optical isomer, pharmaceutically acceptable salt, N-oxide, hydrate or any combination thereof, and a suitable carrier or diluent

In one embodiment, this invention provides a SARM compound, represented by a structure of formula (III), and/or, optical isomer, pharmaceutically acceptable salt, pharmaceutical product, hydrate, N-oxide, or combinations thereof. In another embodiment, this invention provides an optical isomer of the SARM compound. In another embodiment, this invention provides a pharmaceutically acceptable salt of the SARM compound. In another embodiment, this invention provides a hydrate of the SARM compound. In another embodiment, this invention provides an N-oxide of the SARM compound. In another embodiment, this invention provides composition comprising a SARM compound, as described herein, or, in another embodiment, a combination of an optical, isomer, pharmaceutically acceptable salt, hydrate, N-oxide, of the SARM compounds of the present invention. The SARM of the present invention may be isolated from a mixture of the (R) and the (S) isomers, or a racemic mixture comprising an equal amount of the (R) and the (S) isomers. It is well known in the art how to prepare optically-active form of structure (III) (for example, by resolution of the racemic form by recrystallization techniques, by synthesis from optically-active starting materials, by chiral synthesis, or by chromatographic separation using a chiral stationary phase).

The invention includes "pharmaceutically" acceptable salts" of the SARM of this invention, which may be produced, in one embodiment, using an amino-substituted SARM of structure (III) and an organic and inorganic acids, for example, citric acid and hydrochloric acid. Pharmaceutically acceptable salts can be prepared, from the phenolic compounds, in other embodiments, by treatment with inorganic bases, for example, sodium hydroxide. In another embodiment, esters of the phenolic compounds can be made with aliphatic and aromatic carboxylic acids, for example, acetic acid and benzoic acid esters.

The invention also includes N-oxides of the amino substituents of the SARM of the present invention. In another embodiment, this invention further includes hydrates of the SARM compound of structure (III). In one embodiment, "hydrate" includes hemihydrate, monohydrate, dihydrate or trihydrate.

### Selective Androgen Receptor Modulators (SARMS)

Selective androgen receptor modulators (SARMs) are a class of androgen receptor targeting agents (ARTA), which demonstrate androgenic and anabolic activity of a nonsteroidal ligand for the androgen receptor. The novel agent of structure (III) is useful in males for the treatment of a variety of hormone-related conditions such as sexual dysfunction, decreased sexual libido, erectile dysfunction, hypogonadism, sarcopenia, osteopenia, osteoporosis, alterations in cognition and mood, depression, anemia, hair loss, obesity, benign prostate hyperplasia and/or prostate cancer. Further, SARMs are useful for oral testosterone replacement therapy, and imaging prostate cancer. In addition, SARMs are useful in females for the treatment of a variety of hormone-related conditions including, such as sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer.

As contemplated herein, this invention provides a Selective Androgen Receptor Modulator (SARM) compound. SARM compounds, which are useful in preventing and treating muscle wasting disorders and bone related disorders are classified as androgen receptor agonists (AR agonists), partial agonists or androgen receptor antagonists (AR antagonists).

A receptor agonist is a substance which binds receptors and activates them. A receptor partial agonist is a substance which binds receptor and partially activate them. A receptor antagonist is a substance which binds receptors and inactivates them. As demonstrated herein, SARM compounds may, have a tissue-selective effect, wherein, for example, a single agent is an agonist, partial agonist and/or antagonist, depending on the tissue in which the receptor is expressed. For example, SARM compounds may stimulate muscle tissue and concurrently inhibit prostate tissue. SARMs which are useful in treating and preventing muscle wasting disorders are AR agonists, and are, therefore, useful in binding to and activating the AR. SARMs which are AR antagonists, are useful in binding to and inactivating the AR. Assays to determine whether a SARM compound is an AR agonist or antagonist are well known to a person skilled in the art. For example, AR agonistic activity can be determined by monitoring the ability of SARM compounds to maintain and/or stimulate the growth of AR containing tissue such as prostate and seminal vesicles, as measured by weight. AR antagonistic activity can be determined by monitoring the ability of SARM compounds to inhibit the growth of AR containing tissue.
SARM compounds can be classified as partial AR agonist/antagonists. The SARMs are AR agonists in some tissues, to cause increased transcription of AR-responsive genes (e.g. muscle anabolic effect). In other tissues, these compounds serve as competitive inhibitors of testosterone/DHT on the AR to prevent agonistic effects of the native androgens. The term SARM or selective androgen receptor modulator refers, to a compound which modulates androgen receptor activity.

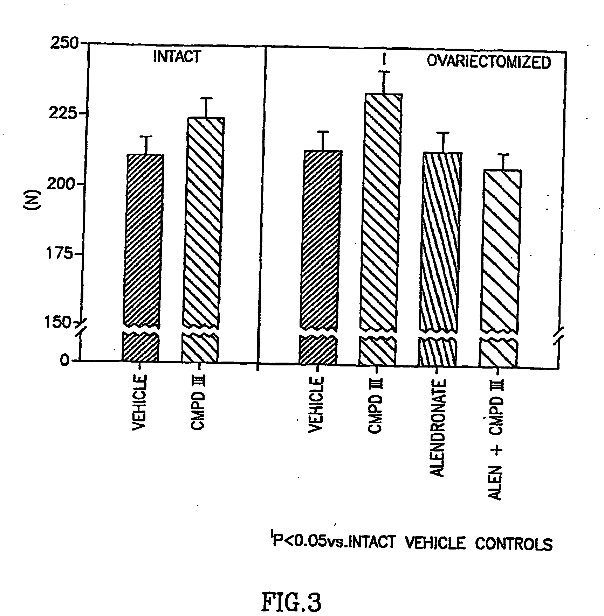

In one instance, the SARM will have antagonist activity in a gonad of a subject, and agonist activity peripherally, such as, for example, in muscle. Such activity was demonstrated herein, in terms of effects on prostate tissue versus that of *levator ani* muscle tissue, as exemplified in Figure 3, 4 or 5.

In one instance, SARM compounds bind reversibly or, in another instance, irreversibly to the androgen receptor. In one instance, SARM compounds bind reversibly to the androgen receptor. In another instance SARM compounds bind irreversibly to the androgen receptor. SARM compounds may contain a functional group (affinity label) that allows alkylation of the androgen receptor (i.e. covalent bond formation). Thus, in this case, the compounds bind irreversibly to the receptor and, accordingly, cannot be displaced by a steroid, such as the endogenous ligands DHI and testosterone.

Modulation of the androgen receptor refers to the ability of the compound to stimulate or enhance signaling through the receptor, and any or, all, downstream effects of receptor signal transduction.

Modulation of the androgen receptor may also refer to the ability of the compound to diminish or abrogate signaling through the receptor, and any or, all, downstream effects of receptor signal transduction.

In another instance, a SARM may interact with a homologue of an androgen receptor. In one instance, the term "homologue of an androgen receptor" refers to structurally or, in another instance, functionally related receptors, whose regulation is desired. In one instance, SARMs may interact with estrogen receptors, or, in another instance, other cell surface molecules which are involved in anabolic pathways, or in another instance steroidogenic pathways, or in another instance, metabolic pathways.

In one embodiment, this invention also provides for a composition comprising the SARM, of this invention.

In one embodiment the composition is a pharmaceutical composition, which, in another embodiment is a pellet, a tablet, a capsule, micronized and non-micronized capsule, a solution, a suspension, an emulsion, an elixir, a gel, a cream, a suppository or a parenteral formulation.

In one embodiment, the micronized capsules comprise particles containing the SARM of this invention, wherein the term "micronized" used herein refers to particles having a particle size is of less than 100 microns, or in another embodiment, less than 50 microns, or in another embodiment, less than 35 microns, or in another embodiment, less than 15 microns, or in another embodiment, less than 10 microns, or in another embodiment, less than 5 microns.

The pharmaceutical compositions may be administered in any effective, convenient manner including, for instance, administration by intravascular (i.v.), intramuscular (i.m.), intranasal (i.n.), subcutaneous (s.c.), sublingual, oral, rectal, intravaginal delivery, or by any means in which the recombinant virus/composition can be delivered to tissue (e.g., needle or catheter). Alternatively, topical administration may be desired for application to mucosal cells, for skin or ocular application. Another method of administration is via aspiration or aerosol formulation.

For administration to mammals, and particularly humans, it is expected that the physician will determine the actual dosage and duration of treatment, which will be most suitable for an individual and can vary with the age, weight and response of the particular individual.

In one embodiment, the compositions for administration may be sterile solutions, or in other embodiments, aqueous or non-aqueous, suspensions or emulsions. In one embodiment, the compositions may comprise propylene glycol, polyethylene glycol, injectable organic esters, for example ethyl oleate, or cyclodextrins. In another embodiment, compositions may also comprise wetting, emulsifying and/or dispersing agents. In another embodiment, the compositions may also comprise sterile water or any other sterile injectable medium.

In one embodiment, the compositions of this invention include, the SARM of this invention or any combination thereof, together with one or more pharmaceutically acceptable excipients.

In one embodiment, "pharmaceutical composition" can mean a therapeutically effective amount of the compound of the present invention together with suitable excipients and/or carriers useful in the methods of this invention. In one embodiment, the compositions will comprise a therapeutically effective amount of the SARM of this invention. In one emboidment, the term "therapeutically effective amount" may refer to that amount that provides a therapeutic effect for a given condition and administration regimen. In one embodiment, such compositions can be administered by any method known in the art.

In one embodiment, the compositions of the present invention are formulated as oral or parenteral dosage forms, such as uncoated tablets, coated tablets, pills, capsules, powders, granulates, dispersions or suspensions. In another embodiment, the compositions of the present invention are formulated for intravenous administration. In another embodiment, the compounds of the present invention are formulated in ointment, cream or gel form for transdermal administration. In another embodiment, the compounds of the present invention are formulated as an aerosol or spray for nasal application. In another embodiment, the compositions of the present invention are formulated in a liquid dosage form. Examples of suitable liquid dosage forms include solutions or suspensions in water, pharmaceutically acceptable fats and oils, alcohols or other organic solvents, including esters, emulsions, syrups or elixirs, solutions and/or suspensions.

Suitable excipients and carriers may be, according to embodiments of the invention, solid or liquid and the type is generally chosen based on the type of administration being used. Liposomes may also be used to deliver the composition. Examples of suitable solid carriers include lactose, sucrose, gelatin and agar. Oral dosage forms may contain suitable binders, lubricants, diluents, disintegrating agents, coloring agents, flavoring agents, flow-inducing agents, and melting agents. Liquid dosage forms may contain, for example, suitable solvents, preservatives, emulsifying agents, suspending agent, diluents, sweeteners, thickeners, and melting agents. Parenteral and intravenous forms should also include minerals and other materials to make them compatible with the type of injection or delivery system chosen. Of course, other excipients may also be used.

The SARM of this invention may be administered at various dosages. In one embodiment, the SARM is administered at a dosage of 0.1 - 200 mg per day. In one embodiment, the SARM is administered at a dose of 0.1 - 10 mg, or in another embodiment, 0.1-25 mg, or in another embodiment, 0.1- 50 mg, or in another embodiment, 0.3-15 mg, or in another embodiment, 0.3 - 30 mg, or in another embodiment, 0.5 - 25 mg, or in another embodiment, 0.5 - 50 mg, or in another embodiment, 0.75 - 15 mg, or in another embodiment, 0.75 - 60 mg, or in another embodiment, 1- 5 mg, or in another embodiment,1- 20 mg, or in another embodiment, 3 -15 mg, or in another embodiment, 30 - 50 mg, or in another embodiment, 30 - 75 mg, or in another embodiment, 100 - 2000 mg.

The SARM of this invention may be administered at various dosages. In one embodiment, the SARM is administered at a dosage of 1 mg. In another embodiment the SARM is administered at a dosage of 5mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg or 100 mg.

In one embodiment, the compounds and compositions of this invention may be used for any of the methods of this invention, as described herein. In one embodiment, use of the SARM or a composition comprising the same, will have utility in inhibiting, suppressing, enhancing or stimulating a desired response in a subject, as will be understood by one skilled in the art. In another embodiment, the compositions may further comprise additional active ingredients, whose activity is useful for the particular application for which the SARM compound is being administered.

This disclosure provides for the use of the SARM compound of this invention, or its optical isomer, pharmaceutically acceptable salt, N-oxide, hydrate or any combination thereof, for 1) treating a bone related disorder; 2) preventing a bone related disorder; 3) suppressing a bone related disorder; 4) inhibiting a bone related disorder; 5) increasing a strength of a bone of a subject; 5) increasing a bone mass in a subject; 6) use for the osteoclastogenesis inhibition. The SARM compound is a compound of formula III, as described herein.

In one instance, the bone related disorder is a genetic disorder, or in another instance, is induced as a result of a treatment regimen for a given disease. For example, and in one instance, the SARM of this invention is useful in treating a bone-related disorder that arises as a result of androgen-deprivation therapy, given in response to prostate carcinogenesis in a subject.

In one instance, the present disclosure provides a use of SARM compound for preventing a bone-related disorder in a subject. In another instance, the present disclosure provides a use of SARM compound for suppressing a bone-related disorder in a subject. In another instance, the present disclosure provides a use of SARM compound for inhibiting a bone-related disorder in a subject. In an embodiment the SARM compound is of formula (III). In another embodiment, the SARM compound is of formula (III) or its optical isomer, pharmaceutically acceptable salt, N-oxide, hydrate or any combination thereof.

In one instance, the bone-related disorder is osteoporosis. In another instance, the bone-related disorder is osteopenia. In another instance, the bone-related disorder is increased bone resorption. In another instance, the bone-related disorder is bone fracture. In another instance, the bone-related disorder is bone frailty. In another instance, the bone-related disorder is a loss of BMD. In another instance, the bone-related disorder is any combination of osteoporosis, osteopenia, increased bone resorption, bone fracture, bone frailty and loss of BMD.

"Osteoporosis" refers, in one instance, to a thinning of the bones with reduction in bone mass due to depletion of calcium and bone protein. In another instance, osteoporosis is a systemic skeletal disease, characterized by low bone mass and deterioration of bone tissue, with a consequent increase in bone fragility and susceptibility to fracture. In osteoporotic patients, bone strength is abnormal, in one instance, with a resulting increase in the risk of fracture. In another instance, osteoporosis depletes both the calcium and the protein collagen normally found in the bone, in one instance, resulting in either abnormal bone quality or decreased bone density. In another instance, bones that are affected by osteoporosis can fracture with only a minor fall or injury that normally would not cause a bone fracture. The fracture can be, in one instance, either in the form of cracking (as in a hip fracture) or collapsing (as in a compression fracture of the spine). The spine, hips, and wrists are common areas of osteoporosis-induced bone fractures, although fractures can also occur in other skeletal areas. Unchecked osteoporosis can lead, in another instance, to changes in posture, physical abnormality, and decreased mobility.

In one instance, the osteoporosis results from androgen deprivation. In another instance, the osteoporosis follows androgen deprivation. In another instance, the osteoporosis is primary osteoporosis. In another instance, the osteoporosis is secondary osteoporosis In another instance, the osteoporosis is postmenopausal osteoporosis. In another instance, the osteoporosis is juvenile osteoporosis. In another instance, the osteoporosis is idiopathic osteoporosis. In another instance, the osteoporosis is senile osteoporosis.

In another instance, the primary osteoporosis is Type I primary osteoporosis. In another instance, the primary osteoporosis is Type II primary osteoporosis.

Osteoporosis and osteopenia are, in another instance, systemic skeletal diseases characterized by low bone mass and microarchitectural deterioration of bone tissue. "Microarchitectural deterioration" refers, in one instance, to thinning of the trabeculae (defined below) and the loss of inter-trabecular connections in bone. In another embodiment, "osteoporosis" is defined as having a BMD 2.5 standard deviations (SD) or more below the young adult mean. In another instance, "osteoporosis" is defined as having a BMC 2.5 SD or more below the young adult mean. In another instance, "osteoporosis" is defined as having a BMD 2.0 SD or more below the young adult mean. In another instance, "osteoporosis" is defined as having a BMC 2.0 SD or more below the young adult mean. In another instance, "osteoporosis" is defined as having a BMD 3.0 SD or more below the young adult mean. In another instance, "osteoporosis" is defined as having a BMC 3.0 SD or more below the young adult mean. Each definition of osteoporosis or osteopenia represents a separate instance of the present invention.

In another instance, "osteoporosis" is defined as having a BMD 2.5 SD below the young adult mean. In another instance, "osteoporosis" is defined as having a BMC 25 SD below the young adult mean. In another instance, "osteoporosis" is defined as having a BMD 2.0 SD below the young adult mean. In another instance, "osteoporosis" is defined as having a BMC 2.0 SD below the young adult mean. In another instance, "osteoporosis" is defined as having a BMD 3.0 SD below the young adult mean. In another instance, "osteoporosis" is defined as having a BMC 3.0 SD below the young adult mean.

Methods for assessing osteoporosis and osteopenia are well know in the art. For example, in one instance a patient's BMD, measured by densitometry and expressed in g/cm², is compared with a "normal value," which is the mean BMD of sex-matched young adults at their peak bone mass, yielding a "T score." In another instance Z-score, the amount of bone loss in a patient is compared with the expected loss for individuals of the same age and sex. In another instance, "osteoporosis" is defined as having a T score 2.5 SD or more below the young adult mean. In another instance, "osteoporosis" is defined as having a Z score 2.5 SD or more below the young adult mean. In another instance, "osteoporosis" is defined as having a T score 2.0 SD or more below the young adult mean. In another instance, "osteoporosis" is defined as having a Z score 2.0 SD or more below the young adult mean. In another instance, "osteoporosis" is defined as having a T score 3.0 SD or more below the young adult mean. In another instance, "osteoporosis" is defined as having a Z score 3.0 SD or more below the young adult mean.

In another instance, "osteoporosis" is defined as having a T score 2.5 SD below the young adult mean. In another instance, "osteoporosis" is defined as having a Z score 2.5 SD below the young adult mean. In another instance, "osteoporosis" is defined as having a T score 2.0 SD below the young adult mean. In another instance, "osteoporosis" is defined as having a Z score 2.0 SD below the young adult mean. In another instance, "osteoporosis" is defined as having a T score 3.0 SD below the young adult mean. In another instance, "osteoporosis" is defined as having a Z score 3.0 SD below the young adult mean. Each definition of osteoporosis represents a separate instance of the present invention.

The term "BMD" is, in one instance, a measured calculation of the true mass of bone. The absolute amount of bone as measured by BMD generally correlates with bone strength and its ability to bear weight By measuring BMD, it is possible to predict fracture risk in the same manner that measuring blood pressure can help predict the risk of stroke.

BMD, in one instance, can be measured by known BMD mapping techniques. In one instance, bone density of the hip, spine, wrist, or calcaneus may be measured by a variety of techniques. The preferred method of BMD measurement is dual-energy x-ray densitometry (DEXA). BMD of the hip, antero-posterior (AP) spine, lateral spine, and wrist can be measured using this technology. Measurement at any site predicts overall risk of fracture, but information from a specific site is the best predictor of fracture at that site. Quantitative computerized tomography (QCT) is also used to measure BMD of the spine. See for example, "Nuclear Medicine: "Quantitative Procedures" by Wahner H W, et al, published by Toronto Little, Brown & Co., 1983, pages 107-132; "Assessment of Bone Mineral Part 1," J Nucl Medicine, pp 1134-1141 (1984); and "Bone Mineral Density of The Radius" J Nucl Medicine 26: 13-39 (1985).

"Osteopenia" refers, in one instance, to having a BMD or BMC between 1 and 25 SD below the young adult mean. In another instance, "osteopenia" refers to decreased calcification or density of bone. This term encompasses, in one instance, all skeletal systems in which such a condition is noted.

In one instance, the term "bone fracture" refers to a breaking of bones, and encompasses both vertebral and non-vertebral bone fractures- The term "bone frailty" refers, in one instance, to a weakened state of the bones that predisposes them to fractures.

In one instance, the bone-related disorder is treated with a SARM compound of this invention, or a combination thereof. In another instance, other bone-stimulating compounds can be provided to a subject, prior to, concurrent with or following administration of a SARM or SARMs of this invention. In one instance, such a bone stimulating compound may comprise natural or synthetic materials.

In one instance, the bone stimulating compound may comprise a bone morphogenetic protein (BMP), a growth factor, such as epidermal growth factor (EGF), a fibroblast growth factor (FGF), a transforming growth factor (TGF-α or TGF-β), an insulin growth factor (IGF), a platelet-derived growth factor (PDGF) hedgehog proteins such as *sonic*, *indian* and *desert* hedgehog, a hormone such as follicle stimulating hormone, parathyroid hormone, parathyroid hormone related peptide, activins, inhibins, *frizzled, frzb* or frazzled proteins, BMP binding proteins such as chordin and fetuin, a cytokine such as IL-3, IL-7, GM-CSF, a chemokine, such as eotaxin, a collagen, osteocalcin or osteonectin.

In another instance, the compositions for use in treating a bone disorder of this invention may comprise a SARM or SARMs of this invention, an additional bone stimulating compound, or compounds, and osteogenic cells. In one instance, an osteogenic cell may be a stem cell or progenitor cell, which may be induced to differentiate into an osteoblast. In another instance, the cell may be an osteoblast

In another instance, nucleic acids which encode bone-stimulating compounds may be administered to the subject, which is to be considered as part of this invention.

In one instance, the osteoporosis, osteopenia, increased bone resorption, bone fracture, bone frailty or loss of BMD are caused by a hormonal disorder, disruption or imbalance. In another instance, these conditions occur independently of a hormonal disorder, disruption or imbalance. Each possibility represents a separate instance of the present invention.

In one instance, the hormonal disorder, disruption or imbalance comprises an excess of a hormone. In another instance, the hormonal disorder, disruption or imbalance comprises a deficiency of a hormone. In one instance, the hormone is a steroid hormone. In another instance, the hormone is an estrogen. In another instance, the hormone is an androgen. In another instance, the hormone is a glucocorticoid. In another instance, the hormone is a cortico-steroid. In another instance, the hormone is Luteinizing Hormone (LH). In another instance, the hormone is Follicle Stimulating Hormone (FSH). In another instance, the hormonal disorder, disruption or imbalance is associated with menopause. In another instance, hormone deficiency is a result of specific manipulation, as a byproduct of treating a disease or disorder in the subject. For example, the hormone deficiency may be a result of androgen depletion in a subject, as a therapy for prostate cancer in the subject

In one instance, the present disclosure provides a use of the SARM compound of structure (III) for increasing a strength of a bone of a subject. In another embodiment, the SARM compound is of formula (III) or its optical isomer, pharmaceutically acceptable salt, N-oxide, hydrate or any combination thereof Thus, increasing a strength of a bone of a subject.

In another instance, the subject has an osteoporosis. In another instance the osteoporosis is hormonally induced.

In one instance, the present disclosure provides a use of the SARM compound of formula (III) for increasing a bone mass of a subject. In another embodiment, the SARM compound is of formula or its, optical isomer, pharmaceutically acceptable salt, N-oxide, hydrate or any combination thereof, or a composition comprising the same.

In another instance, the subject has osteoporosis. In another instance the osteoporosis is hormonally induced. In another instance the subject has sarcopenia or cachexia. In another instance the present disclosure provides for increasing a bone mass in the subject, which is a cortical bone mass. In another instance the bone mass is trabecular bone mass. In another instance the bone mass is a cancellous bone mass.

In one instance, the present disclose provides use of the SARM compound of formula (III) for promoting bone formation. In another embodiment, the SARM compound is of formula or its optical isomer, pharmaceutically acceptable salt, N-oxide, hydrate or any combination thereof, or a composition comprising the same.

In another instance, the SARM compound of formula (III) stimulates or enhances osteoblastogenesis. In another instance, the said SARM compound inhibits osteoblast prolification.

In one instance, the present disclosure provides for bone formation via osteoblast stimulation or enhanced proliferation. In one instance, the term "osteoblast" refers to cell which participates in bone-formation. In one instance, osteoblast involvement in bone formation may form the tissue and deposit minerals therein, giving bone its strength. In another instance, the present disclosure provides for bone formation via suppression of osteoclast induction, or in another instance, activity. In one instance, the term "osteoclast" refers to a cell which participates in bone remodeling, and in particular in bone resorption.

In one instance, bone diseases or disorders are treated by the methods of the present disclosure via stimulation of bone formation. In another instance, the treatments the present disclosure provide for maintenance of bone mass. Bone mass is maintained by a balance between the activity of osteoblasts that form bone and osteoclasts that break it down. In one instance, the compounds and methods of the present disclosure provide a means whereby such a balance is maintained.

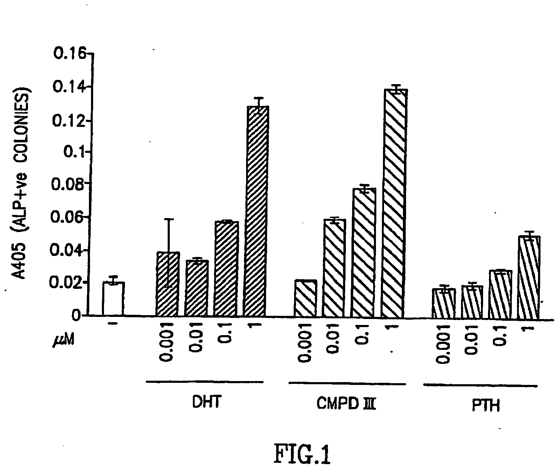

Figures 1-2 demonstrate that SARM compound III induced differentiation of bone morrow cell to osteoblasts yet inhibited osteoclast induction, indicating direct effects of SARMs on both osteoblasts and osteoclasts, which would be useful in increasing bone mass in osteoporotic patients.

In one instance, the present disclosure, provides use of the SARM compound of this invention, or its optical isomer, pharmaceutically acceptable salt, N-oxide, hydrate or any combination thereof, for 1) treating a muscle wasting disorder, 2) preventing a muscle wasting disorder, 3) treating, preventing, suppressing, inhibiting or reducing muscle loss due to a muscle wasting disorder, 4) treating, preventing, inhibiting, reducing or suppressing muscle wasting due to a muscle wasting disorder, and/or 5) treating, preventing, inhibiting, reducing or suppressing muscle protein catabolism due to a muscle wasting disorder. In one embodiment the SARM compound is a compound oF formula (III) as described herein. In another embodiment, the invention provides a composition comprising the SARM compound III for use in the methods as described herein.

In one instance, the present disclosure provides a use of SARM compound III for treating a subject suffering from a muscle wasting disorder. In another embodiment, the SARM compound is of formula or its optical isomer, pharmaceutically acceptable salt, N-oxide, hydrate or any combination thereof, or a composition comprising the same. Thus, treating a subject suffering from a muscle wasting disorder.

In another instance, the use of a SARM compound for treating a subject suffering from a muscle wasting disorder includes administering a pharmaceutical composition including the SARM compound. In another instance, the administering step includes intravenously, intraarterially, or intramuscularly injecting to said subject said pharmaceutical composition in liquid form; subcutaneously implanting in said subject a pellet containing said pharmaceutical composition; orally administering to said subject said pharmaceutical composition in a liquid or solid form; or topically applying to the skin surface of said subject said pharmaceutical composition.

A muscle is a tissue of the body that primarily functions as a source of power. There are three types of muscles in the body: a) skeletal muscle -the muscle responsible for moving extremities and external areas of the bodies; b) cardiac muscle - the heart muscle; and c) smooth muscle - the muscle that is in the walls of arteries and bowel.

A wasting condition or disorder is defined herein as a condition or disorder that is characterized, at least in part, by an abnormal, progressive loss of body, organ or tissue mass. A wasting condition can occur as a result of a pathology such as, for example, cancer, or an infection, or it can be due to a physiologic or metabolic state, such as disuse deconditioning that can occur, for example, due to prolonged bed rest or when a limb is immobilized, such as in a cast. A wasting condition can also be age associated The loss of body mass that occurs during a wasting condition can be characterized by a loss of total body weight, or a loss of organ weight such as a loss of bone or muscle mass due to a decrease in tissue protein.

In one instance, "muscle wasting" or "muscular wasting", used herein interchangeably, refer to the progressive loss of muscle mass and/or to the progressive weakening and degeneration of muscles, including the skeletal or voluntary muscles which control movement, cardiac muscles which control the heart, and smooth muscles. In one instance, the muscle wasting condition or disorder is a chronic muscle wasting condition or disorder. "Chronic muscle wasting" is defined herein as the chronic (i.e. persisting over a long period of time) progressive loss of muscle mass and/or to the chronic progressive weakening and degeneration of muscle.

The loss of muscle mass that occurs during muscle wasting can be characterized by a muscle protein breakdown or degradation, by muscle protein catabolism. Protein catabolism occurs because of an unusually high rate of protein degradation, an unusually low rate ofprotein synthesis, or a combination of both. Protein catabolism or depletion, whether caused by a high degree of protein degradation or a low degree of protein synthesis, leads to a decrease in muscle mass and to muscle wasting. The term "catabolism" has its commonly known meaning in the art, specifically an energy burning form of metabolism.

Muscle wasting can occur as a result of a pathology, disease, condition or disorder. In one embodiment, the pathology, illness, disease or condition is chronic. In another embodiment, the pathology, illness, disease or condition is genetic. In another embodiment, the pathology, illness, disease or condition is neurological. In another embodiment, the pathology, illness, disease or condition is infectious. As described herein, the pathologies, diseases, conditions or disorders for which the compounds and compositions of the present invention are administered are those that directly or indirectly produce a wasting (i.e. loss) of muscle mass, that is a muscle wasting disorder.

In one instance, muscle wasting in a subject is a result of the subject having a muscular dystrophie; muscle atrophy; X-linked spinal-bulbar muscular atrophy (SBMA), cachexia; malnutrition, tuberculosis, leprosy, diabetes, renal disease, chronic obstructive pulmonary disease (COPD), cancer, end stage renal failure, sarcopenia, emphysema, osteomalacia, or cardiomyopathy.

In another instance, the muscle wasting disorder is due to infection with enterovirus, Epstein-Barr virus, herpes zoster, HIV, trypanosomes, influenze, coxsackie, rickettsia, trichinella, schistosoma or mycobacteria..

The muscular dystrophies are genetic diseases characterized by progressive weakness and degeneration of the skeletal or voluntary muscles that control movement. The muscles of the heart and some other involuntary muscles are also affected in some forms of muscular dystrophy. The major forms of muscular dystrophy (MD) are: duchenne muscular dystrophy, myotonic dystrophy, duchenne muscular dystrophy, becker muscular dystrophy, limb-girdle muscular dystrophy, facioscapulhumeral muscular dystrophy, congenital muscular dystrophy, oculopharyngeal muscular dystrophy, distal muscular dystrophy and emery-dreifuss muscular dystrophy.

Muscular dystrophy can affect people of all ages. Although some forms first become apparent in infancy or childhood, others may not appear until middle age or later. Duchenne MD is the most common form, typically affecting children. Myotonic dystrophy is the most common of these diseases in adults.

Muscle atrophy (MA) is characterized by wasting away or diminution of muscle and a decrease in muscle mass. For example, Post-Polio MA is a muscle wasting that occurs as part of the post-polio syndrome (PPS). The atrophy includes weakness, muscle fatigue, and pain.

Another type of MA is X-linked spinal-bulbar muscular atrophy (SBMA - also known as Kennedy's Disease). This disease arises from a defect in the androgen receptor gene on the X chromosome, affects only males, and its onset is in adulthood. Because the primary disease cause is an androgen receptor mutation, androgen replacement is not a current therapeutic strategy. There are some investigational studies where exogenous testosterone propionate is being given to boost the levels of androgen with hopes of overcoming androgen insensitivity and perhaps provide an anabolic effect. Still, use of supraphysiological levels of testosterone for supplementation will have limitations and other potentially serious complications.

Cachexia is weakness and a loss of weight caused by a disease or as a side effect of illness. Cardiac cachexia, i.e. a muscle protein wasting of both the cardiac and skeletal muscle, is a characteristic of congestive heart failure. Cancer cachexia is a syndrome that occurs in patients with solid tumors and hematological malignancies and is manifested by weight loss with massive depletion of both adipose tissue and lean muscle mass.

Cachexia is also seen in acquired immunodeficiency syndrome (AIDS), human immunodeficiency virus (HIV)-associated myopathy and/or muscle weakness/wasting is a relatively common clinical manifestation of AIDS. Individuals with HIV-associated myopathy or muscle weakness or wasting typically experience significant weight loss, generalized or proximal muscle weakness, tenderness, and muscle atrophy.

Sarcopenia is a debilitating disease that afflicts the elderly and chronically ill patients and is characterized by loss of muscle mass and function. Further, increased lean body mass is associated with decreased morbidity and mortality for certain muscle-wasting disorders. In addition, other circumstances and conditions are linked to, and can cause muscle wasting disorders. For example, studies have shown that in severe cases of chronic lower back pain, there is paraspinal muscle wasting.

Muscle wasting is also associated with advanced age. It is believed that general weakness in old age is due to muscle wasting. As the body ages, an increasing proportion of skeletal muscle is replaced by fibrous tissue. The result is a significant reduction in muscle power, performance and endurance.

Long term hospitalization due to illness or injury, or disuse deconditioning that occurs, for example, when a limb is immobilized, can also lead to muscle wasting. Studies have shown that in patients suffering injuries, chronic illnesses, bums, trauma or cancer, who are hospitalized for long periods of time, there is a long-lasting unilateral muscle wasting, with a consequent decrease in body mass.

Injuries or damage to the central nervous system (CNS) are also associated with muscle wasting disorders. Injuries or damage to the CNS can be, for example, caused by diseases, trauma or chemicals. Examples are central nerve injury or damage, peripheral nerve injury or damage and spinal cord injury or damage.

In another instance, muscle wasting may be a result of alcoholism, and may be treated with the compounds and compositions of the invention.

In one instance, the present disclosure provides a use of SARM compound of formula (III) for preventing a muscle wasting disorder in a subject. The SARM compound is of formula (III) or its optical isomer, pharmaceutically acceptable salt, N-oxide, hydrate or any combination thereof. In another instance, the administering comprises administering a pharmaceutical composition comprising said SARM and/or its optical isomer, metabolite, pharmaceutically acceptable salt, pharmaceutical product, hydrate, N-oxide, or any combination thereof, and a pharmaceutically acceptable carrier. Thus, preventing a muscle wasting disorder in a subject

In one instance, the invention provides a use of SARM compound of formula (III) treating a muscle-wasting conditions associated with chronic illness. The SARM compound is of formula (III) or its optical isomer, pharmaceutically acceptable salt, N-oxide, hydrate or any combination thereof or a composition comprising the same. In another instance, the use of said SARM compound is orally administered to said subject

In one instance, the present disclosure provides a use of the SARM compounds III for preventing a muscle wasting disorder in a subject, in another instance, suppressing a muscle wasting disorder in a subject, in another instance inhibiting a muscle wasting disorder in a subject, in another instance reducing the incidence of a muscle wasting in a subject. The SARM compound is of formula (III) or its optical isomer, pharmaceutically acceptable salt, N-oxide, hydrate or any combination thereof or a composition comprising the same.

In another instance, the present disclosure provides for the use of the SARM compound of this invention, or its optical isomer, pharmaceutically acceptable salt, N-oxide, hydrate or any combination thereof, or a composition comprising the same, in treating, preventing, suppressing, inhibiting or reducing the incidence of a muscle wasting disorder in a subject.

In another instance, the present disclosure provides for the use of the SARM of this invention, or its optical isomer, pharmaceutically acceptable salt, N-oxide, hydrate or any combination thereof, or a composition comprising the same, in increasing muscle performance, muscle size, muscle strength, or any combination thereof in a subject.

In another instance, the SARM and compositions of this invention are useful in promoting or speeding recovery following a surgical procedure.

In one instance, the present disclosure provides a use of the SARM compound III or reducing a fat mass in a subject. The SARM compound is of formula (III) or its isomer, derivative, pharmaceutically acceptable salt, pharmaceutical product, polymorph, crystal, impurity, N-oxide, hydrate or any combination thereof, or a composition comprising the same.

In another instance, the present disclosure provides for the use of the SARM compound of this invention, having the structure of formula (III) or its, optical isomer, pharmaceutically acceptable salt, N-oxide, hydrate or any combination thereof, or a composition comprising the same, in treating obesity or diabetes associated with a metabolic syndrome in a subject

In another instance, the subject has a hormonal imbalance, disorder, or disease. In another instance the subject has menopause.

In one instance, the present disclosure provides a use of the SARM compound III for increasing a lean mass in a subject. The SARM compound is of formula III or its optical isomer, metabolite, derivative, pharmaceutically acceptable salt, N-oxide, hydrate or any combination thereof. Thus, increasing a lean mass in a subject.

In another instance the subject has a hormonal imbalance, disorder, or disease. In another instance the subject has menopause.

Figures 3-7 demonstrate that Compound III is anabolic yet minimally androgenic, thus such compounds may be useful in treating patient groups in which androgens were contraindicated in the past. Compound III was shown to stimulate muscle growth, whether in the presence or absence of testosterone while exerting anti-proliferative effects on the prostate, thus, in one instance, the SARM of this invention restore lost muscle mass in patients with sarcopenia or cachexia.

In one instance, the SARM of this invention is administered intravenously, via injecting the pharmaceutical composition in liquid form to the subject. In another instance, the SARM of this invention is administered intra-arterially, via injecting the pharmaceutical composition in liquid form to the subject. In another instance, the SARM of this invention is administered intramuscularly via injecting the pharmaceutical composition in liquid form to the subject. In another instance, the SARM of this invention is administered subcutaneously via implanting a pellet containing the pharmaceutical composition in the subject. In another instance the SARMs of this invention are administered orally via administering the pharmaceutical composition in a liquid or solid form to the subject. In another instance the SARMs of this invention are administered topically via applying the pharmaceutical composition to the skin surface of the subject.

The present disclosure provides, in one instance, the use of compound III in a safe and effective method for treating, preventing, suppressing, inhibiting or reducing loss of muscle and/or muscle protein catabolism due to muscle wasting. The invention is useful, in another instance, in treating a subject suffering from a muscle wasting disorder, or in another instance in treating a bone related disorder. In one embodiment, the subject is a mammalian subject.

In another instance, the present disclosure relates to the use of SARM compound III in a method of preventing, suppressing, inhibiting or reducing the incidence of obesity in a subject, comprising the step of administering to the subject the selective androgen receptor modulator (SARM) of this invention and/or its, optical isomer, pharmaceutically acceptable salt, hydrate, N-oxide, or any combination thereof, in an amount effective to prevent, suppress, inhibit or reduce the incidence of obesity in the subject.

In one instance, the SARM compound of the present invention alters the levels of leptin in a subject. In another instance, the SARM compound decreases the levels of leptin. In another embodiment, the SARM compound of the present invention increases the levels of leptin in a subject. Leptin is known to have an effect on appetite on weight loss in obese mice, and thus has been implicated in obesity.

The SARM of this invention, in one instance, affects circulating, or in another instance, tissue levels of leptin. In one instance, the term 'level/s of leptin' refers to the serum level of leptin. As contemplated herein, the SARM compound of the present invention has an effect on leptin *in*-*vitro* and *in*-*vitro*. Leptin levels can be measured by methods known to one skilled in the art, for example by commercially available ELISA kits. In addition, Leptin levels may be determined in *in-vitro* assays, or in *in-vivo* assays, by any method known to a person skilled in the art.

Since leptin is implicated in controlling appetite, weight loss, food intake, and energy expenditure, modulating and/or controlling the levels of leptin is a useful therapeutic approach in treating preventing, inhibiting or reducing the incidence of obesity in subjects suffering from obesity. Modulating the level of leptin can result in a loss of appetite, a reduction of food intake, and an increase in energy expenditure in the subject, and thus may contribute to the control and treatment of obesity.

The term "obesity" is defined, in one instance, as an increase in body weight beyond the limitation of skeletal and physical requirement, as the result of excessive accumulation of fat in the body.

The term "obesity-associated metabolic disorder" refers, in one instance, to a disorder which results from, is a consequence of, is exacerbated by or is secondary to obesity. Non-limiting examples of such a disorder are osteoarthritis, Type II diabetes mellitus, increased blood pressure, stroke, and heart disease.

The term "osteoarthritis" refers, in another instance, to a non-inflammatory degenerative joint disease occurring chiefly in older people, characterized by degeneration of the articular cartilage, hypertrophy of bones and the margins and changes in the synovial membrane. It is accompanied, in other instances, by pain and stiffness, particularly after prolonged activity.

The term "diabetes", in one instance, refers to a relative or absolute lack of insulin leading to uncontrolled carbohydrate metabolism. Most patients can be clinically classified as having either insulin-dependent diabetes mellitus (IDDM or Type-I diabetes) or non-insulin-dependent diabetes mellitus (NIDDM or Type-II diabetes).

The term "increased blood pressure" or "hypertension" refers, in other instances, to a repeatedly high blood pressure above 140 over 90 mmHg. Chronically-elevated blood pressure can cause blood vessel changes in the back of the eye, thickening of the heart muscle, kidney failure, and brain damage.

The term "stroke" refers, in other instances, to damage to nerve cells in the brain due to insufficient blood supply often caused by a bursting blood vessel or a blood clot. The term "heart disease", in other instances refers to a malfunction in the heart normal function and activity, including heart failure

In addition, androgens have recently been shown to be involved in commitment of mesenchymal pluripotent cells into myogenic lineage and to block differentiation into adipogenic lineage (Singh et al., Endocrinology, 2003, Jul 24). Accordingly, selective androgen receptor modulator compounds can be useful in methods of blocking adipogenesis, and/or altering stem cell differentiation, as described herein.

In another instance, the present disclosure relates to the use of SARM compound III in a method of promoting, increasing or facilitating weight loss in a subject, comprising the step of administering to the subject the selective androgen receptor modulator (SARM) of this invention and/or its optical isomer, pharmaceutically acceptable salt, hydrate, N-oxide, or any combination thereof, in an amount effective to promote, increase or facilitate weight loss in the subject.

In another instance, the present disclosure relates to the use of compound III in a method of decreasing, suppressing, inhibiting or reducing appetite of a subject, comprising the step of administering to the subject the selective androgen receptor modulator (SARM) of this invention and/or its optical isomer, pharmaceutically acceptable salt, hydrate, N-oxide, or any combination thereof, in an amount effective to decrease, suppress, inhibit or reduce the appetite of the subject.

In another instance, the present disclosure relates to the use of SARM compound III in a method of altering the body composition of a subject, comprising the step of administering to the subject the selective androgen receptor modulator (SARM) of this invention and/or its, optical isomer, pharmaceutically acceptable salt, hydrate, N-oxide, or any combination thereof, in an amount effective to alter the body composition of the subject. In one instance, altering the body composition comprises altering the lean body mass, the fat free body mass of the subject, or a combination thereof.

In another instance, the present disclosure relates to the use of SARM compound III in a method of altering lean body mass or fat free body mass of a subject, comprising the step of administering to the subject the selective androgen receptor modulator (SARM) of this invention and/or its, isomer, pharmaceutically acceptable salt, hydrate, N-oxide, or any combination thereof, in an amount effective to alter the lean body mass or fat free body mass of the subject.

In another instance, the present disclosure relates to the use of SARM compound III in a method of converting fat to lean muscle in a subject, comprising the step of administering to the subject the selective androgen receptor modulator (SARM) of this invention and/or its, optical isomer, pharmaceutically acceptable salt, hydrate, N-oxide, or any combination thereof, in an amount effective to convert fat to lean muscle in the subject.

In another instance, the present disclosure relates to the use of SARM compound III in a method of treating an obesity-associated metabolic disorder in a subject, comprising the step of administering to the subject the selective androgen receptor modulator (SARM) of this invention and/or its optical isomer, metabolite, pharmaceutically acceptable salt, hydrate, N-oxide, or any combination thereof, in an amount effective to treat the obesity-associated metabolic disorder in the subject

In another instance, the present disclosure relates to the use of SARM compound III in a method of preventing, suppressing, inhibiting or reducing an obesity-associated metabolic disorder in a subject, comprising the step of administering to the subject the selective androgen receptor modulator (SARM) of this invention and/or its, isomer, pharmaceutically acceptable salt, hydrate, N-oxide, or any combination thereof, in an amount effective to prevent, suppress, inhibit or reduce the obesity-associated metabolic disorder in the subject.

In one instance, the obesity-associated metabolic disorder is hypertension. In another instance, the disorder is osteoarthritis. In another instance, the disorder is Type II diabetes mellitus. In another instance, the disorder is increased blood pressure. In another instance, the disorder is stroke. In another embodiment, the disorder is heart disease.

In another instance, the present disclosure relates to the use of SARM compound III in a method of decreasing, suppressing, inhibiting or reducing adipogenesis in a subject, comprising the step of administering to the subject the selective androgen receptor modulator (SARM) of this invention and/or its, optical isomer, pharmaceutically acceptable salt, hydrate, N-oxide, or any combination thereof, in an amount effective to decrease, suppress, inhibit or reduce adipogenesis in the subject.

In another the present disclosure relates to the use of SARM compound III in a method of altering stem cell differentiation in a subject, comprising the step of administering to the subject a selective androgen receptor modulator (SARM) of this invention and/or its optical isomer, pharmaceutically acceptable salt, hydrate, N-oxide, or any combination thereof, in an amount effective to alter stem cell differentiation in the subject.

In another instance, the present disclosure relates to the use SARM compound III in a method of attering the level of leptin in a subject, comprising the step of administering to the subject a selective androgen receptor modulator (SARM) of this invention and/or its optical isomer, pharmaceutically acceptable salt, hydrate, N-oxide or any combination thereof, in an amount effective to alter the level of leptin in the subject. In one embodiment, altering the level of leptin comprises decreasing the level of leptin in the subject.

In another instance, the present disclosure relates to the use of SARM compound III in method of decreasing, suppressing, inhibiting or reducing the level of leptin in a subject, comprising the step of administering to the subject the selective androgen receptor modulator (SARM) of this invention and/or its optical isomer, pharmaceutically acceptable salt, hydrate, N-oxide, or any combination thereof, in an amount effective to decrease, suppress, inhibit or reduce the level of leptin in the subject.

In one instance, the SARM that is useful in a) treating, preventing, suppressing, inhibiting, or reducing obesity; b) promoting, increasing or facilitating weight loss; c) decreasing, suppressing, inhibiting or reducing appetite; d) altering the body composition; e) altering lean body mass or fat free body mass; f) converting fat to lean muscle; g) treating, preventing, suppressing, inhibiting, or reducing an obesity-associated metabolic disorder, for example hypertension, osteoarthritis. Type II diabetes mellitus, increased blood pressure, stroke, or heart disease; h) decreasing, suppressing, inhibiting or reducing adipogenesis; i) altering stem cell differentiation; and/or j) altering the level of leptin, is a compound represented by the structure of formula (III).

In one instance, the SARM of this invention finds utility in treating or halting the progression of, or treating symptoms of diabetes. In another instance, the SARM of this invention is useful in treating co-morbidities related to diabetes. These conditions include: hypertension, cerebrovascular disease, atherosclerotic coronary artery disease, macular degeneration, diabetic retinopathy (eye disease) and blindness, cataracts-systemic inflammation (characterized by elevation of inflammatory markers such as erythrocyte sedimentation rate or C-reactive protein), birth defects, pregnancy related diabetes, pre-ecclampsia and hypertension in pregnancy, kidney disease (renal insufficiency renal failure), nerve disease (diabetic neuropathy), superficial and systemic fungal infections, congestive heart failure, gout/hyperuricemia, obesity, hypertriglyceridemia, hypercholesterolemia, fatty liver disease (non-alcoholic steatohepatitis, or NASH), and diabetes-related skin diseases such as Necrobiosis Lipoidica Diabeticorum (NLD), Blisters of diabetes (Bullosis Diabeticorum), Eruptive Xanthomatosis, Digital Sclerosis, Disseininated Granuloma Annulare, and Acanthosis Nigricans.

In one instance, the present disclosure provides the use of SARM compound III in method for a) treating, preventing, suppressing inhibiting atherosclerosis b) treating, preventing, suppressing inhibiting liver damage due to fat deposits comprising the step of administering to the subject the selective androgen receptor modulator (SARM) of this invention and/or its, optical isomer, pharmaceutically acceptable salt, hydrate, N-oxide, or any combination thereof, or a composition comprising the same, in an amount effective to treat, prevent or inhibit atherosclerosis and liver damage due to fat deposit.

In one instance, the SARM is useful in a) treating, preventing, suppressing, inhibiting, or reducing atherosclerosis; b) treating, preventing, suppressing inhibiting liver damage due to fat deposits.

In one instance atherosclerosis refers to a slow, complex disease that may begin with damage to the innermost layer of the artery. In another instance the causes of damage to the arterial wall may include a) elevated levels of cholesterol and in the blood; b) high blood pressure; c) tobacco smoke d) diabetes. In another instance, the condition is treatable in a smoker, despite the fact that tobacco smoke may greatly worsen atherosclerosis and speed its growth in the coronary arteries, the aorta and arteries in the legs. Similarly, in another instance, compound of formula (III) disclosure may be useful in treating subjects with a family history of premature cardiovascular disease who have an increased risk of atherosclerosis.

In one instance, liver damage due to fat deposits refer to the build-up of fat in the liver cells forming a Fatty Liver which may be associated with or may lead to inflammation of the liver. This can cause scarring and hardening of the liver. When scarring becomes extensive, it is called cirrhosis. In another instance the fat accumulates in the liver as obesity. In another instance fatty liver is also associated with diabetes mellitus, high blood triglycerides, and the heavy use of alcohol. In another instance fatty Liver may occur with certain illnesses such as tuberculosis and malnutrition, intestinal bypass surgery for obesity, excess vitamin A in the body, or the use of certain drugs such as valproic acid (trade names: Depakene/Depakote) and corticosteroids (cortisone, prednisone). Sometimes fatty liver occurs as a complication of pregnancy.

In one instance, the methods of use in treating a subject are where the subject is a human, and in another instance, where the subject is male, or in another instance, where the subject is female.

In another instance the present disclosure provides for the use of the SARM of this invention, or a composition comprising the same, in promoting or suppressing spermatogenesis in a male subject. Some SARMs exhibit, *inter-alia*, androgenic activity, which in turn stimulates spermatogenesis. In other instance, the SARMs exhibit antagonist activity in the gonads of a subject, which in turn, may suppress spermatogenesis. In one instance, the SARM may therefore be used as a contraceptive.

The following examples are presented in order to more fully illustrate the characteristics of selective Androgen Receptor Modulator (SARM) compound of formula III and the preferred embodiments of the invention. They should in no way, however, be construed as limiting the broad scope of the invention.

### EXAMPLES

### EXAMPLE 1:

### Effects of Selective Androgen Receptor Modulator (SARM) compound III on Progenitor Cell Differentiation To Osteoblasts and Osteoclasts.

### Materials and Methods:

### Chemicals

Compound III, THT and PTH were prepared at concentrations ranging from 1nM-1 µM.

### Animals

Four month old female rats were sacrificed by euthanasia and the femurs were excised from the animals. The femurs were cleaned off any muscle and connective tissues and were stored on ice in Minimum Essential Medium (MEM) with penicillin, Streptomycin and Fungizone until the cells were cultured.

### Bone Marrow Cell Culture

All cell culture materials were obtained from Invitrogen (Carisbad, CA). The femurs were first rinsed in 70% ethanol and were washed three times with 5 ml each of penicillin and streptomycin. Both the ends of the femurs were snapped and the bone marrow cells were flushed with 15 ml of MEM with penicillin, Streptomycin and Fungizone into a 50 ml conical tube and stored on ice. The same procedure was performed with all the femurs. The bone marrow cells and were pooled were centrifuged at 1000 rpm for 5 min in a clinical centrifuge. The cells were resuspended in MEM without phenol red supplemented with 10% charcoal stripped serum, penicillin, streptomycin and fungizone. The cells were triturated through a 22g needle, counted under microscope and were plated at 1.5 million cells per well of a 6 well plate in MEM without phenol red supplemented with 15% charcoal stripped serum, penicillin streptomycin, 300 ng/ml fungizone, 0.28 mM Ascorbic acid and 10 mM β-glycerophosphate to differentiate towards fibroblast/osteoblast lineage and at 2.5 million cells per well of a 24 well plate in MEM without phenol red supplemented with 10% charcoal stripped serum, penicillin streptomycin and 300 ng/ml fungizone to differentiate towards osteoclast lineage. The medium was changed on day 2 and the cells were treated with the indicated hormone. Osteoclast cultures were carried out in the presence of 50 ng RANK Ligand and 10 ng GM-CSF to induce osteoclastogenesis. Medium was completely changed every third day for osteoclast cultures. For fibroblast cultures, half the culture medium was changed every third day to leave the growth factors secreted by the cells.

### Staining of Cells

At the end of 12 days, the cells were fixed in 10% buffered formalin for fibroblast cultured and in 4% formaldehyde in PBS for osteoclast cultures. The fibroblasts were stained for alkaline phosphatase activity and the O.D. at 405 nm was measured using a spectrophotometer as described earlier. Osteoclasts were stained for Tartarate Resistant Acid Phosphatase Activity (TRAP) and cells having 2 or more nuclei were counted under the microscope and plotted as indicated earlier.

### Results

### SARMs are potent inducers of differentiation of bone marrow cells towards the ostcoblast and osteoclast lineage

Androgens exert anabolic effects on bone and lack of androgens under conditions such as androgen deprivation therapy in prostate cancer and in old age have clearly indicated the benefits of androgens as a bone protective hormone. However, the use of ectopic androgen is limited due to its side effects and also due to the risk of conversion of androgens to estrogens.

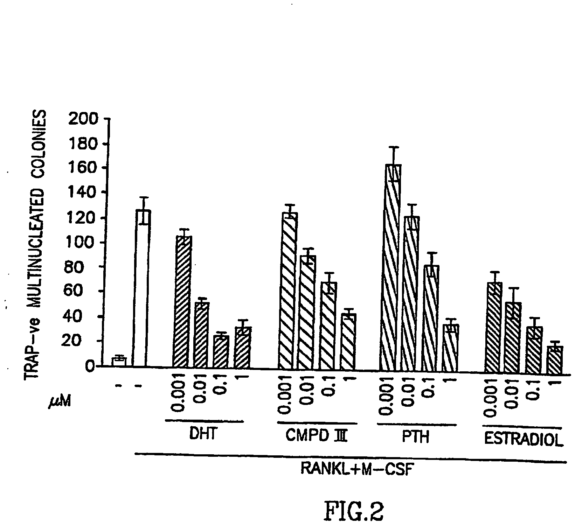

In order to determine whether a SARM could be therapeutic yet obviate the above side-effects, various selective androgen receptor modulators (SARMs) were evaluated in terms of their ability to have bone protective effects, with fewer side effects, as seen with the parent hormone. The efficacy of Di-hydro testosterone (DHT) and Parathyroid hormone (PTH) were compared to a SARM, Compound III in terms of their ability to differentiate primary rat bone marrow cells towards the osteoblast and the osteoclast lineage (Figures 1 and 2). Bone marrow cells from rats were cultured in the presence or absence of the above indicated hormones for 12 days in culture medium and were evaluated in terms of their differentiation towards osteoblast or osteoclast lineage.

DHT and Compound III all increased differentiation of primary bone marrow cells toward the osteoblast lineage as measured by alkaline phosphatase (ALP) activity of the cells (Fig. 1). At 1 µM concentration, DHT and the SARM induced the ALP activity comparably whereas at lower concentrations of 100 nM and 10 nM Compound III showed better induction than the DHT. PTH, another bone anabolic hormone induced the ALP staining only at higher concentration but not at lower concentrations.

Fig. 2 shows a clear increase in the number of TRAP positive multinucleated osteoclasts, when cells were incubated in the presence of RANK ligand and GM-CSF. Treatment of cells with DHT or SARM significantly inhibited RANK ligand and GM-CSF- induced TRAP positive multinucleated osteoclast proliferation. PTH inhibited induction at higher concentrations, however, at lower concentrations, PTH increased the number of TRAP positive osteoclasts. Estradiol inhibited osteoclastogenesis, at all dosages evaluated.

### EXAMPLE 2:

### SARM BONE EFFECTS ALONE AND IN COMBINATION WITH THE ANTIRESORPTIVE AGENT, ALENDRONATE

### Materials and Methods:

Sixty female, virgin, intact Sprague-Dawley rats were obtained from Charles River Laboratories (Wilmington, MA) and aged to 23 wks. The animals were housed 2-3 per cage and acclimated to a 12-h light/dark cycle. Food (7012C LM-485 Mouse/Rat Sterilizable Diet, Harlan Teklad, Madison, WI) and water were provided *ad libitum.* The Institutional Animal Care and Use Committee of the University of Tennessee reviewed and approved the animal protocol for this study.

Sham surgeries or ovariectomies were performed on Day 0. The study was comprised of six treatment groups as follows: (1) intact + vehicle, (2) intact + COMPOUND III, (3) OVX + vehicle, (4) OVX + COMPOUND III, (5) OVX + alendronate (6) OVX + alendronate + COMPOUND III. Doses (200 L) were administered daily via oral gavage in a vehicle of DMSO:PEG300 (10:90) beginning on Day 1. Animals were sacrificed on Day 45 of the study. Femurs were removed, cleared of soft tissue, and stored in saline soaked gauze at -20°C until analysis. Nine animals died during the course of the study. These deaths were attributed to surgical complications arising from the ovariectomies and technical errors during oral dosing (i.e., dosing solution delivered into the lungs). Dose groups are listed in Table 1.

**Table 1. Treatment groups**

| **Group** | **Gonadal Status** | **Treatment** | **Dose** | **Animals/group** |
|---|---|---|---|---|
| 1 | Intact | Vehicle | N/A | 9 |
| 2 | Intact | COMPOUND III | 3 mg/day | 9 |
| 3 | OVX | Vehicle | N/A | 7 |
| 4 | OVX | COMPOUND III | 3 mg/day | 8 |
| 5 | OVX | Alendronate | 1 mg/day | 10 |
| | | Alendronate | + 1 mg/day | + 3 |
| 6 | OVX | COMPOUND III | mg/day | 8 |

The left femurs were sent to SkeleTech Inc. (Bothell, WA) for biomechanical strength (three point bending) and pQCT analysis. A Stratec XCT RM and associated software (Stratec Medizintechnik GmbH, Pforzheim, Germany. Software version 5.40 C) were used for the pQCT analysis. The femur was analyzed at both the mid-shaft and distal regions. The mid-shaft analysis was performed on the region at 50% of the length of the fernur. The distal analysis was performed on the region at 20% of the length of the femur starting at the distal end. One 0.5 mm slice perpendicular to the long axis of the femur was used for analysis. Total bone mineral content, total bone area, total bone mineral density, cortical bone mineral content conical bone area, cortical bone mineral density, cortical thickness, periosteal perimeter (circumference) and endosteal perimeter were determined at the mid-shaft of the femur. As the distal femur, total bone mineral content, total bone area, total bone mineral density, trabecular bone mineral content trabecular bone area and trabecular bone mineral density were determined. Following pQCT analisys femoral Strength was determined by a three-point bending test. The anterior posterior diameter (APD) (unit mm) at the midpoint of the femoral shaft was measured with an electronic caliper. The fermur was placed on the lower supports of a three-point bending fixture with the anterior side of the femur facing downward in an Instron Mechanical Testing Machine (Instron 4465 retrofitted to 5500)(Camon, MA). The length (L) between the lower supports was set to 14 mm. The upper loading device was aligned to the center of the femoral shaft. The load was applied at a constant displacement rate of 6 mm/min until the femur broke. The mechanical testing machine directly measured the maximum load (Fₙ) (unitN), stiffness (S) (units:N/mm), and energy absorbed (W) (unit mJ). The axial area moment of inertia (I) (unit mm) was calculated by the software during the pQCT analysis of the femoral mid-shaft. Sress (σ) (units:N/mm²), elastic modulus (E) (unitMpa), and tonghness (T) (units:mJ/m³) were calculated by the following formulas: strest: σ= (Fᵤ * L *(a/2)) / (4* I); elastic modulus: B = S*L^{J}/(48*I); and toughness: T = 3*W*(APD/2)²/(L*I).
Statistical analysis was performed by Student's T-test. P-values of less than 0.05 were considered as statistically significant differences.

### Results:

Femoral maximum load was determined by 3-point bending of the femur. Results are shown in Figure 3. No differences were observed between the intact vehicle (210 N) and the OVX vehicle (212 N) control groups. We observed trends in the COMPOUND III treated groups with maximum load increasing to 224 and 233 newtons in the intact and OVX groups, respectively. The alendronate (213 N) and alendronate + COMPOUND III (207N) groups were not different from controls.

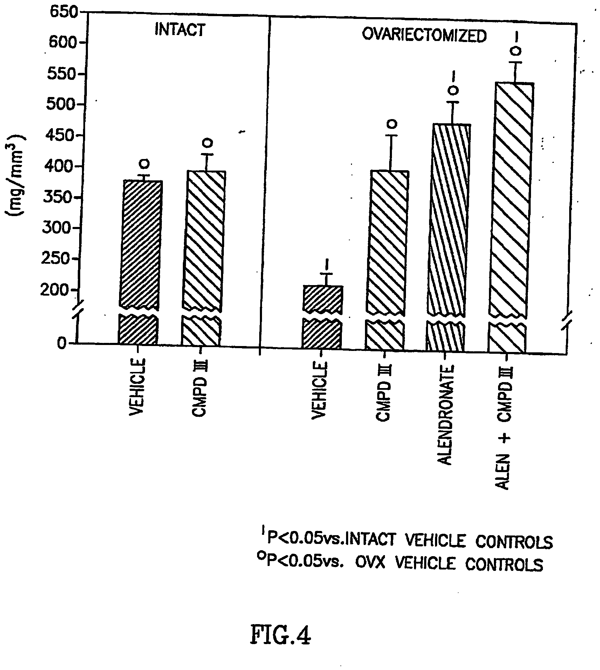

Trabecular bone mineral density was analyzed by pQCT at the distal femur. Results are shown in Figure 4. We observed significant trabecular bone loss following OVX. Trabecular bone density decreased from 379 to 215 mg/mm³ in the intact and OVX vehicle control groups, respectively. In intact animals treated with COMPOUND III, we observed a slight increase in trabecular bone density to 398 mg/mm³. In OVX animals treated with COMPOUND III, we observed a significant increase over the OVX vehicle control group to 406 mg/mm³. Alendronate increased trabecular bone density to 480 mg/mm³. The combination therapy of Alendronate and COMPOUND III showed additive effects increasing trabecular bone density to 552 mg/mm³.

### EXAMPLE 3:

### Androgenic & Anabolic Activity in Intact and ORX Rats

### Materials and Methods:

Male Sprague-Dawley rats weighing approximately 200g were purchased from Harlan Bioproducts for Science (Indianapolis, IN). The animals were maintained on a 12-h light/dark cycle with food (7012C LM-485 Mouse/Rat Sterilizable Diet, Harlan Teldad, Madison, WI) and water available *ad libitum.* The animal protocol was reviewed and approved by the Institutional Animal Care and Use Committee of the University of Tennessee. Anabolic and androgenic activity of Compound III in intact animals was evaluated, and the dose response in acutely orchidectomized (ORX) animals was evaluated as well. Regenerative effects of Compound III in chronically (9 days) ORX rats was also assessed.

The compound was weighed and dissolved in 10% DMSO (Fisher) diluted with PEG 300 (Acros Organics, NJ) for preparation of the appropriate dosage concentrations. The animals were housed in groups of 2 to 3 animals per cage. Intact and ORX animals were randomly assigned to one of seven groups consisting of 4 to 5 animals per group. Control groups (intact and ORX) were administered vehicle daily. Compound III was administered via oral gavage at doses of 0.01, 0.03, 0.1, 0.3, 0.75, and 1 mg/day to both intact and ORX groups.

Castrated animals (on day one of the study) were randomly assigned to dose groups (4-5 animals/group) of 0.01, 0.03, 0.1, 0.3, 0.75, and I mg/day, for dose-response evaluation. Dosing began nine days post ORX and was administered daily via oral gavage for fourteen days. The animals were sacrificed under anesthesia (ketamine/xyalzine, 87:13 mg/kg) after a 14-day dosing regimen, and body weights were recorded. In addition, ventral prostate, seminal vesicles, and levator ani muscle were removed, individually weighed, normalized to body weight, and expressed as a percentage of intact control. Student's T-test was used to compare individual dose groups to the intact control group. Significance was defined *a priori* as a P-value < 0.05. As a measure of androgenic activity, ventral prostate and seminal vesicle weights were evaluated, whereas levator ani muscle weight was evaluated as a measure of anabolic activity. Blood was collected from the abdominal aorta, centrifuged, and sera were frozen at -80°C prior to determination of serum hormone levels. Serum luetinizing hormone (LH) and follicle stimulating hormone (FSH) concentrations were determined by the University of Virginia Center for Research in Reproduction Ligand Assay and Analysis Core (NICHD (SCCPRR) Grant U54-HD28934).

### Results:

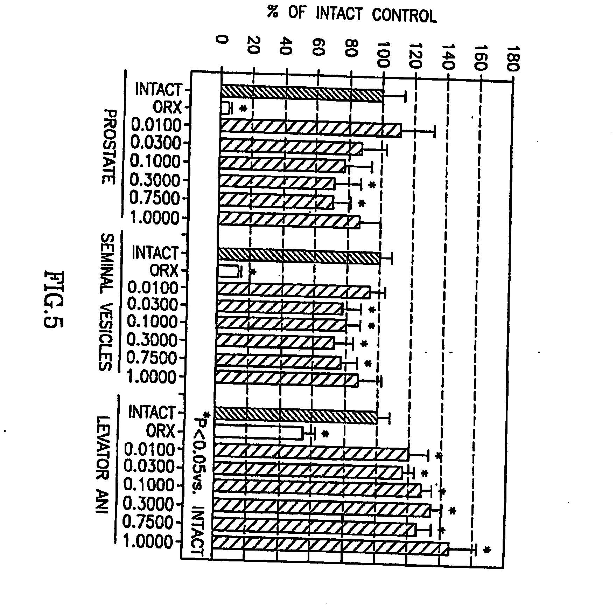

Prostate weights following Compound III treatment were 111% ± 21%, 88% ± 15%, 77% ± 17%, 71% ± 16%, 71% :b 10%, and 87% ± 13% of intact controls following doses of 0.01, 0.03, 0.1, 0.3, 0.75, and 1 mg/day, respectively (Figure 5). Similarly, seminal vesicle weights decreased to 94% ± 9%, 77% ± 11%, 80% ± 9%, 73% ± 12%, 77% ± 10%, and 88% ± 14% of intact controls following doses of 0.01, 0.03, 0.1, 0.3, 0.75, and I mg/day, respectively. Significant increases were seen in levator ani muscle weights of sham animals, however, in all dose groups, when compared to intact controls. The levator ani muscle weights were 120% ± 12%, 116% ± 7%, 128% ± 7%, 134% ± 7%, 125% ± 9%, and 146% ± 17% of intact controls corresponding to 0.01, 0.03, 0.1, 0.3, 0.75, and 1.0 mg/day dose groups, respectively. The results are presented graphically in Figure 5.

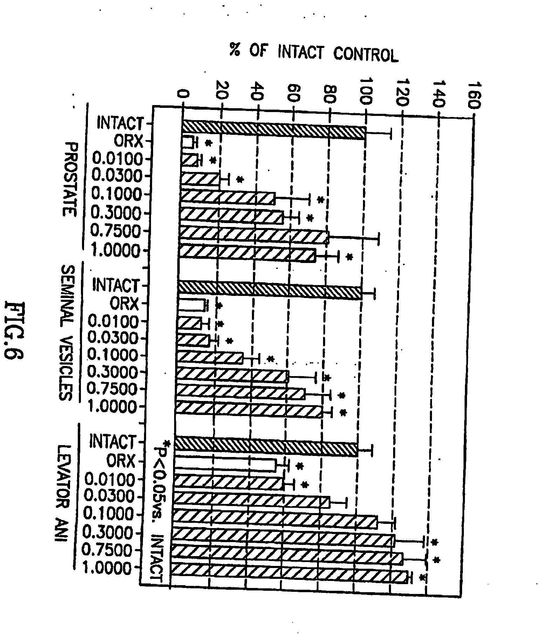

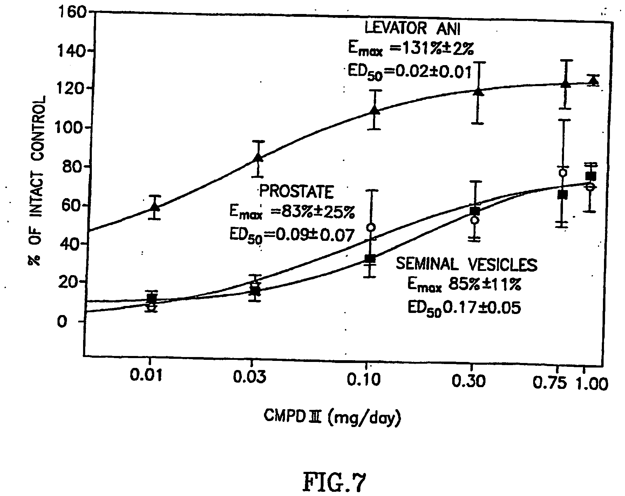

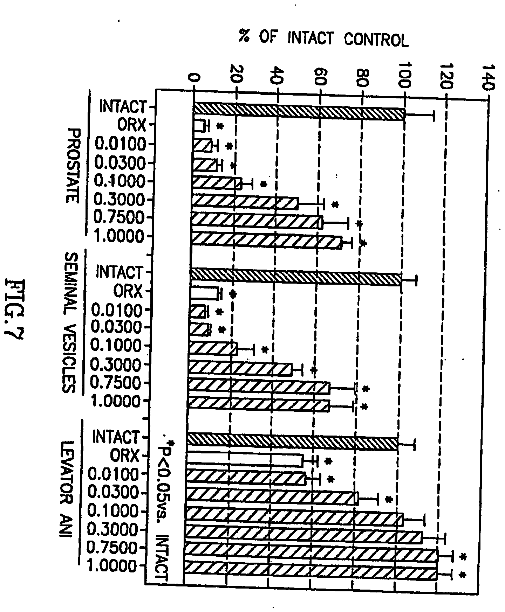

Compound III partially maintained prostate weight following orchidectomy. Prostate weight in vehicle treated ORX controls decreased to 5% ± 1% of intact controls. At doses of 0.01, 0.03, 0.1, 0.3, 0.75, and 1.0 mg/day, Compound III maintained prostate weights at 8% ± 2%, 20% ± 5%, 51% ± 19%, 56% ± 9%, 80% ± 28%, and 74 ± 12.5% of intact controls, respectively. In castrated controls, seminal vesicle weight decreased to 13% ± 2% of intact controls. Compound III partially maintained seminal vesicle weights in ORX animals. Seminal vesicle weights from drug treated animals were 12% ± 4%, 17% ± 5%, 35% ± 10%, 61% ± 15%, 70% ± 14%, and 80% ± 6% of intact controls, following doses of 0.01, 0.03, 0.1, 0.3, 0.75, and 1.0 mg/day, respectively. In ORX controls the levator ani muscle weight decreased to 55% ± 7% of intact controls. We observed an anabolic effect in the levator ani muscle of Compound III treated animals. Compound III fully maintained levaor ani muscle weights at doses > 0.1 mg/day. Doses > 0.1 mg/day resulted in significant increases in levator ani weight compared to that observed in intact controls. Levator ani muscle weights as a percentage of intact controls were 59% ± 6%, 85% ± 9%, 112% ± 10%,122% ± 16%, 127 ± 12%, and 129.66 ± 2% for the 0.01, 0.03, 0.1, 0.3, 0.75, and 1.0 mg/day dose groups, respectively. Results are graphically presented in Figure 6. Eₘₐₓ and ED₅₀ values were determined in each tissue by nonlinear regression analysis in WinNonlin® and presented in Figure 7. Eₘₐₓ values were 83% ± 25%, 85% ± 11%, and 131% ± 2% for prostate, seminal vesicles, and levator ani, respectively. The ED₅₀ in prostate, seminal vesicles, and levator ani was 0.09 ± 0.07, 0.17 ± 0.05, and 0.02 ± 0.01 mg/day, respectively.

### Serum Hormone Analysis

Serum LH and FSH data for the animals are presented in Table 1. LH decreased in a dose-dependent manner in both intact and castrated animals. Following doses > 0.1 mg/day, LH levels were below the limit of quantitation (0.07 ng/mL). The 0.1 mg/day dose in ORX animals returned LH levels back to those seen in intact controls. Similar effects were observed with FSH. In intact animals, a significant decrease in FSH levels was observed with the 0.75 and 1 mg/day doses. In ORX animals, a dose-dependent decrease in FSH levels was observed. Doses of Compound III > 0.1 mg/day in ORX animals returned FSH levels to those of intact controls.

**Table 1. Serum LH and FSH levels from animals in Arm 1 and Arm2. "P<0.05 vs. Intact Controls. ^{b}P<0.05 vs. ORX Controls.**

| | Luetinizing Hormone | | Follicle Stimulating Hormone | |
|---|---|---|---|---|
| Compound III | Intact | ORX | Intact | ORX |
| (mg/day) | (ng/ml) | (ng/ml) | (ng/ml) | (ng/ml) |
| Vehicle | 0.281 ± 0.126^{b} | 9.66 ± 1.13^{a} | 6.40 ± 1 58^{b} | 43.45 ± 4.97^{a} |
| 0.01 | 0 195 ± 0 106^{b} | 8.45 ± 2.44^{a} | 5 81 ± 031^{b} | 36.23 ± 7 75^{a} |
| 0.03 | 0.176 ± 0.092^{b} | 4.71 ± 1.72^{a,b} | 5 74 ± 0.78^{b} | 40.15 ± 3.33^{a} |
| 0.1 | 0.177 ± 0.058^{b} | 0.778 ± 0.479^{b} | 6.60 ±1.06^{b} | 20.69 ± 3.52^{a,b} |
| 0 3 | < LOQ | < LOQ | 5.32 ± 1.80^{b} | 8.73 ± 2.25^{b} |
| 0.75 | < LOQ | < LOQ | 4 30 ± 0.62^{a,b} | 7.19 ± 1.11^{b} |
| 1 | < LOQ | < LOQ | 4.38 ± 042^{a,b} | 6 33 ± 0.70^{b} |

### Androgenic & Anabolic Activity Following Delayed Dosing

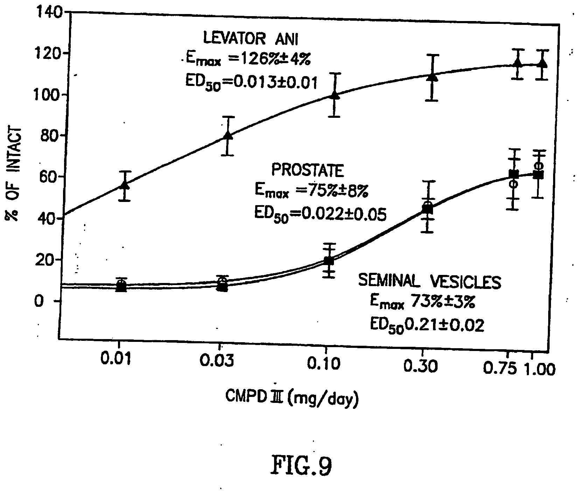

Compound III partially restored both prostate and seminal vesicle weight in ORX animals. Prostates were restored to 9% ± 3%, 11% ± 3%, 23% ± 5%, 50% ± 13%, 62% ± 12%, and 71% ± 5%, while seminal vesicles were restored 7% ± 1%, 9% ± 1%, 23% ± 8%, 49% ± 5%, 67% ± 12%, and 67% ± 11% of intact controls for the 0.01, 0.03, 0.1, 0.3, 0.75, and 1.0 mg/day dose groups, respectively. Compound III fully restored levator ani muscle weight at doses > 0.1 mg/day. Levator ani muscle weights were restored to 56% ± 7%, 82% ± 9%, 103% ± 11%, 113% ± 11%, 121% ± 7%, and 120% ± 7% corresponding to doses of 0.01, 0.03, 0.1, 0.3, 0.75, and 1.0 mg/day, respectively. Results are presented graphically in Figure 8. Eₘₐₓ and ED₅₀ values were determined in each tissue by nonlinear regression analysis in WinNonlin® and presented in Figure 9. Emax values were 75% ± 8%, 73% ± 3%, and 126% ± 4% for prostate, seminal vesicles, and levator ani, respectively. The ED₅₀ in prostate, seminal vesicles, and levator ani was 0.22 ± 0.05, 0.21 ± 0.02, and 0.013 ± 0.01 mg/day, respectively.

### EXAMPLE 4:

### Pharmacokinetic characterization of the novel oral anabolic SARM compound III: The first analysis in healthy male volunteers

### Materials and Methods

Cohorts of a maximum of 12 healthy male volunteers were dosed at each dose level (9 active, 3 placebo) in a randomized, double-blind study design. Eight cohorts were recruited (aged 18-45 years) and each cohort received one single oral dose corresponding to either 1, 3, 10, 30 or 100 mg compound III (or placebo of equal volume of PEG300) in solution, or 3 or 30 mg in experimental capsules. The effect of micronization (i.e. particle size reduction) was investigated on the pharmacokinetics of compound III in the 30 mg solid oral dosage form. Samples for pharmacokinetic assessment of parent drug were taken for up to 72 hours following dosing.

### Results

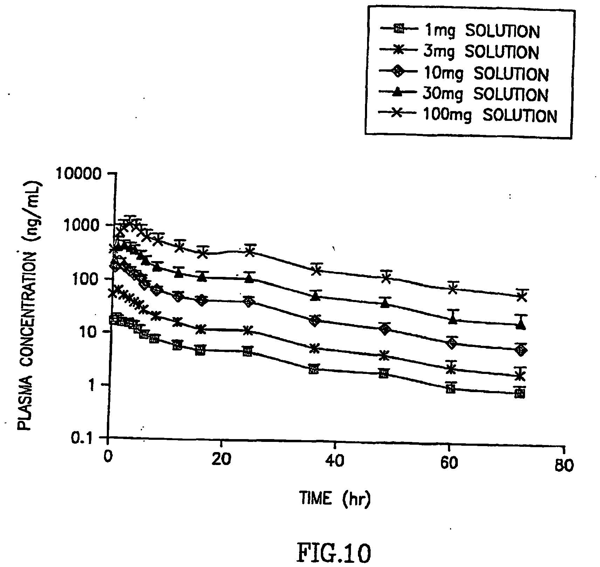

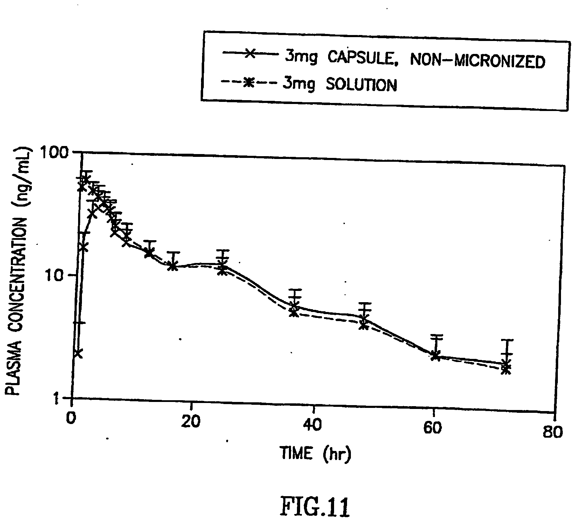

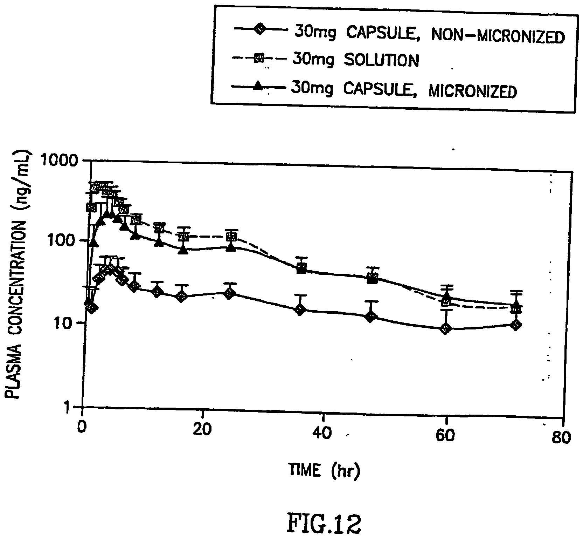

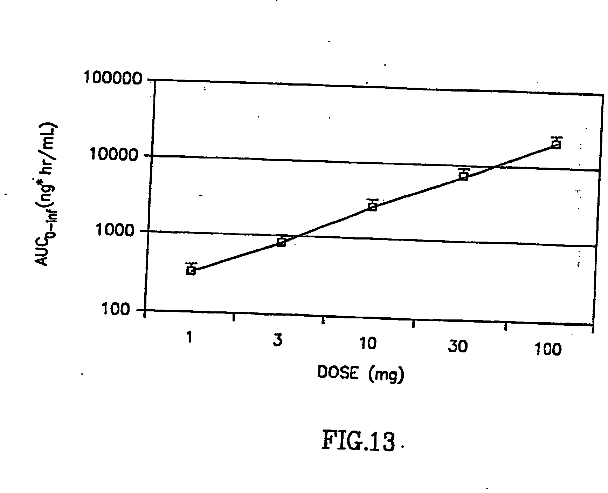

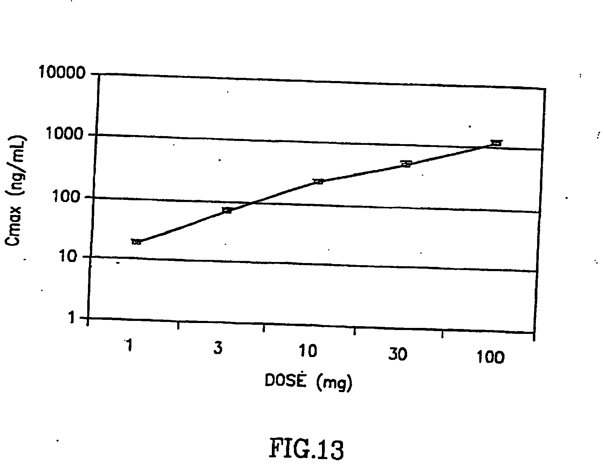

Doses of compound III in PEG300-based solutions at 1, 3, 10, 30 and 100 mg were rapidly absorbed from the gastrointestinal tract. All dose levels resulted in plasma compound III concentrations that were quantifiable through the last time point collected (72 hours) (Figure 10 -12). Exposure (Cmax and AUC) to compound III increased with increasing dose and was linear for solutions over the dose range 1 to 100 mg. Tₘₐₓ was achieved between 0.8 and 2.3 hours (median value = 1.0 hours) for compound III in solution, and between 3.2 and 3.9 hours following the solid oral formulations (Figure 13 & 14). The terminal elimination half-life ranged from 19 to 22 hours (median value = 20 hours) for 1-100 mg solutions and the 3 mg capsule, and was increased with the 30 mg capsules to 27 and 31 hours for micronized and non-micronized, although not significantly (p>0.1). Oral clearance was inversely associated with half-life, with the 30 mg non-micronized capsule exhibiting the longest half-life and the lowest clearance compared to the other dosage forms and amounts. The 3 mg non-micronized capsule and solution were equally bioavailable, but at the higher dose (30 mg) micronization improved oral bioavailability (p<0.05) (Figure 12). As suggested by a consistent second peak over the elimination phase of the drug, it is possible that enterohepatic recirculation through the hepatobiliary system plays a role in redistribution of parent drug.

### EXAMPLE 5 (comparative)

### ANABOLIC AND ANDROGENIC ACTIVITY OF SARMs

Materials. The SARMS are synthesized essentially in accordance with methods as described in United States Patent Application Publication Number 2004/0014975 A1. Alzet osmotic pumps (model 2002) are purchased from Alza Corp. (Palo Alto, CA).

The SARMs tested will comprise the following: 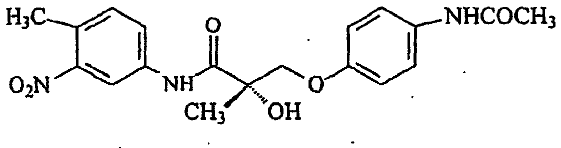 And 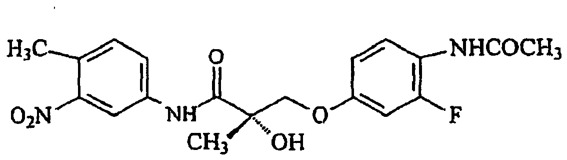 And their activity will be compared to that of: 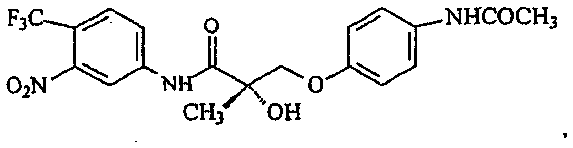 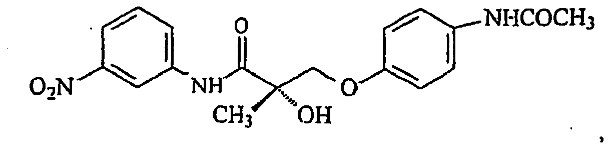 and

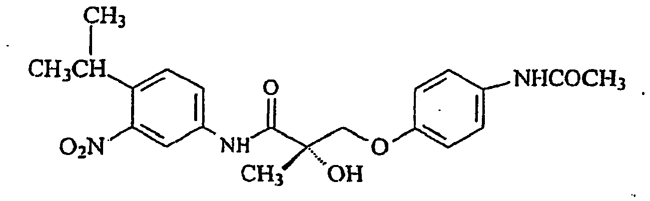

*Study Design.* Immature male Sprague-Dawley rats, weighing 90 to 100g, are randomly distributed into groups, with at least 5 animals per group. One day prior to the start of drug treatment, animals are individually removed from the cage, weighed and anesthetized with an intraperitoneal dose of ketamine/xylazine (87/13 mg/kg; approximately 1 mL per kg). When appropriately anesthetized (i.e., no response to toe pinch), the animals' ears are marked for identification purposes. Animals are then placed on a sterile pad and their abdomen and scrotum washed with betadine and 70% alcohol. The testes are removed via a midline scrotal incision, with sterile suture being used to ligate supra-testicular tissue prior to surgical removal of each testis. The surgical wound site is closed with sterile stainless steel wound clips, and the site cleaned with betadine. The animals are allowed to recover on a sterile pad (until able to stand) and then returned to their cage.

Twenty-four hours later, animals are re-anesthetized with ketamine/xylazine, and an Alzet osmotic pump(s) (model 2002) containing the SARM compound is placed subcutaneouly in the scapular region. Osmotic pumps contain the appropriate treatment (as described in Example 3) dissolved in polyethylene glycol 300 (PEG300). Osmotic pumps are filled with the appropriate solution one day prior to implantation. Animals are monitored daily for signs of acute toxicity to drug treatment (e.g., lethargy, rough coat).

After 14 days of drug treatment, rats are anesthetized with ketamine/xylazine. Animals are sacrificed by exsanguination under anesthesia. A blood sample is collected by venipuncture of the abdominal aorta, and submitted for complete blood cell analysis. A portion of the blood is placed in a separate tube, centrifuged at 12,000g for I minute, and the plasma layer removed and frozen at - 20°C. The ventral prostates, seminal vesicles, levator ani muscle, liver, kidneys, spleen, lungs, and heart are removed, cleared of extraneous tissue, weighed, and placed in vials containing 10% neutral buffered formalin. Preserved tissues are subjected to histopathological analysis.

For data analysis, the weights of all organs are normalized to body weight, and analyzed for any statistical significant difference by single-factor ANOVA. The weights of prostate and seminal vesicle are used as indexes for evaluation of androgenic activity, and the levator ani muscle weight is used to evaluate the anabolic activity.

Testosterone propionate (TP), at increasing doses, is used as the positive control of anabolic and androgenic effects. Effects of particular compounds may thus be compared to that ofTP.

The weights of prostate, seminal vesicle, and levator ani muscle in castrated, vehicle-treated rats are expected to decrease significantly, due to the ablation of endogenous androgen production. Exogenous administration of testosterone propionate, an androgenic and anabolic steroid, are expected to increase the weights of prostate, seminal vesicle, and levator ani muscle in castrated rats in a dose-dependent manner. The SARMs will be comparatively evaluated for their effect on the weights of prostate, seminal vesicle, and levator ani muscle in castrated animals. Compounds which show lower potency and intrinsic activity in increasing the weights of prostate and seminal vesicle, but a greater potency and intrinsic activity in increasing the weight of levator ani muscle, will be considered to be poorly androgenic yet anabolic, and represent compounds which would be useful in therapy of, for example, prostate cancer, or for treating side effects associated with current therapies for prostate cancer, such as, for example, androgen deprivation therapy.

### EXAMPLE 6

### SARM REDUCTION OF CHOLESTEROL LEVELS

### Materials and Methods

One hundred Sprague Dawley rats (50 male and 50 female) were divided into five groups (n=10 per gender per group), representing vehicle only (PEG300:40% Cavasol® [75/25 (v/v)]), and four dose groups of Compound III. Animals were administered Compound III once daily by oral gavage according to their most recent body weight with doses of either 0, 3, 10, 30 or 100 mg/kg. During the study period, rats had access to water and a standard laboratory diet of Harlan Taldad Rodent Chow *ad libitum.* After 28 consecutive days of dosing, animals were fasted overnight, blood samples were collected and processed to yield serum. Serum levels of total cholesterol were determined using an automated laboratory assay method.

### Results

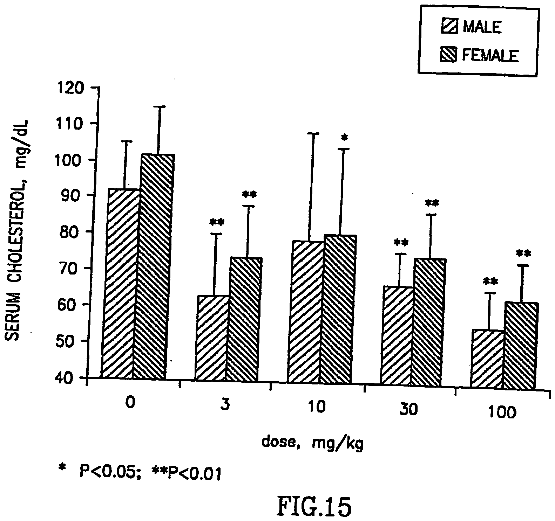

The male and female rats in the vehicle only group (0 mg/kg) had serum total cholesterol values of 92±13.5 and 102±13 mg/dL respectively. These values are considered within the normal historical range for the testing laboratory. Daily oral doses of Compound III at or above 3 mg/kg caused a significant reduction in total cholesterol levels in both male and female rats. At 3 mg/kg, compared to vehicle control animals, an approximate 30% reduction in total cholesterol was noted where males and females had 63±17.4 and 74±14.2 mg/dL respectively. Although a slightly greater effect was noted at the highest dose group (100 mg/kg per day), in general, a dose-response relationship was not observed in the reduction of total cholesterol levels in the Sprague Dawley rat. Results are presented graphically in Figure 15.

The effect of SARMs in causing acute toxicity, as gauged by diagnostic hematology tests and visual examination of animals receiving treatments will be assessed, as will suppression of luteinizing hormone (LH) or follicle stimulating hormone (FSH), as described in Example 4 hereinabove.

## Claims

1. A selective androgen receptor modulator (SARM) compound, represented by a structure of formula (III):

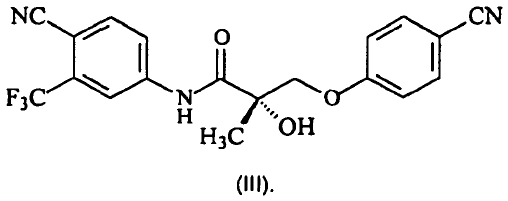

2. The selective androgen receptor modulator (SARM) compound according to claim 1, wherein said compound is its optical isomer, pharmaceutically acceptable salt, N-oxide, hydrate or any combination thereof.

3. A pharmaceutical composition comprising the SARM compound of claim 1 or 2 and a suitable carrier or diluent.

4. The composition of claim 3, further comprising alendronate.

## Patentansprüche

1. Selektive Androgenrezeptormodulator- (SARM)-Verbindung mit einer Struktur der Formel (III)

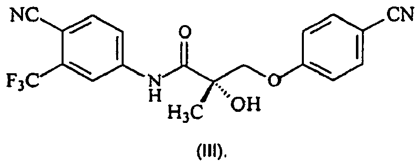

2. Selektive Androgenrezeptormodulator- (SARM)-Verbindung nach Anspruch 1, bei der die Verbindung ihr optisches Isomer, pharmazeutisch annehmbares Salz, N-Oxid, Hydrat oder jegliche Kombination davon ist.

3. Pharmazeutische Zusammensetzung, die die SARM-Verbindung nach Anspruch 1 oder 2 und einen geeigneten Träger oder ein geeignetes Verdünnungsmittel enthält.

4. Zusammensetzung nach Anspruch 3, die ferner Alendronat umfasst.

## Revendications

1. Composé modulateur sélectif des récepteurs aux androgènes (SARM), représenté par la structure de formule (III) :

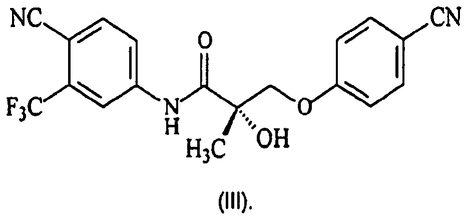

2. Composé modulateur sélectif des récepteurs aux androgènes (SARM) selon la revendication 1, dans lequel ledit composé est son isomère optique, son sel pharmaceutiquement acceptable, son N-oxyde, son hydrate ou une quelconque combinaison de ceux-ci.

3. Composition pharmaceutique comprenant le composé SARM de la revendication 1 ou 2, et un support ou diluant adéquats.

4. Composition de la revendication 3, comprenant également de l'alendronate.
